(19) Europäisches Patentamt
European Patent Office
Office européen des brevets

(11) Numéro de publication : **0 081 425**
**B1**

(12) **FASCICULE DE BREVET EUROPÉEN**

(45) Date de publication du fascicule du brevet : **18.09.85**

(21) Numéro de dépôt : **82402189.3**

(22) Date de dépôt : **30.11.82**

(51) Int. Cl.⁴ : **A 61 K 31/18**// C07C143/822, C07C93/14, C07C143/70, C07C43/225, C07C43/205, C07C145/00, C07C147/10

(54) **Compositions pharmaceutiques présentant des propriétés normolipémiantes et contenant des composés ayant une structure du type benzènesulfonamides n-substitués.**

(30) Priorité : **02.12.81 FR 8122598**

(43) Date de publication de la demande : **15.06.83 Bulletin 83/24**

(45) Mention de la délivrance du brevet : **18.09.85 Bulletin 85/38**

(84) Etats contractants désignés : **AT BE CH DE GB IT LI LU NL SE**

(56) Documents cités :
**EP-A- 0 029 974**
**GB-A- 512 460**
**US-A- 3 337 592**

(73) Titulaire : **CHOAY S.A.**
**48, Avenue Théophile-Gautier**
**F-75782 Paris Cedex 16 (FR)**

(72) Inventeur : **Fournier, Jean-Paul**
**10, rue Fontenay**
**F-78000 Versailles (FR)**
Inventeur : **Roger, Pierre**
**6, rue Paul Valéry**
**F-78423 Montigny Les Bretonneux (FR)**
Inventeur : **Choay, Patrick**
**7, Cour Jasmin**
**F-75016 Paris (FR)**

(74) Mandataire : **Gutmann, Ernest et al**
**Cabinet Plasseraud 84, rue d'Amsterdam**
**F-75009 Paris (FR)**

**EP 0 081 425 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention est relative à des compositions pharmaceutiques présentant des propriétés normolipémiantes et qui contiennent, à titre de substance active, des composés présentant une structure de base du type benzènesulfonamides N-substitués ou un sel physiologiquement acceptable de ces composés.

L'invention vise également le procédé de préparation des susdits médicaments ainsi que leurs sels.

D'après le brevet GB n° 512 460, on connaît des p-aminobenzène sulfonalcanolamides de formule :

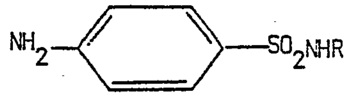

dans laquelle R représente un groupe alcanol ayant 3 ou 4 atomes de carbone et 1 ou 2 groupes hydroxyle, l'un des atomes de carbone étant un atome de carbone secondaire attaché soit à un groupe hydroxy, soit à un atome d'azote.

Ces composés monosubstitués en para par un groupe $NH_2$ sont présentés comme étant efficaces pour le traitement d'infections streptocoques et autres infections.

D'après le brevet US n° 3 337 592, on connaît des composés de formule :

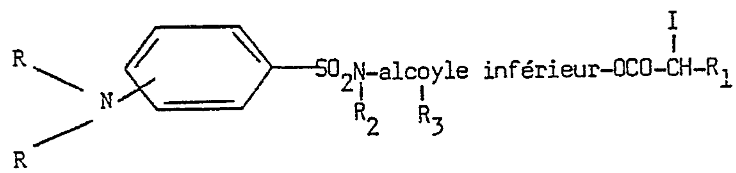

dans laquelle R représente un atome d'hydrogène, un alcoyle inférieur, un alcanoyle inférieur ou un alcoylphényle inférieur, $R_1$ représente un atome d'hydrogène ou un alcoyle inférieur, $R_2$ représente un atome d'hydrogène, un alcoyle inférieur ou le radical :

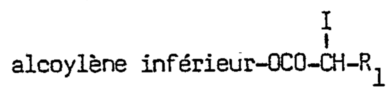

et $R_3$ représente un atome d'hydrogène, un alcoyle inférieur ou OCOCHRI.

Les structures caractéristiques de ces composés sont notamment la présence d'un groupe ester et d'un atome d'iode sur la chaîne ainsi que la présence d'un radical NRR sur le noyau.

Ces composés sont des agents antibactériens et possèdent une activité inhibitrice de la croissance tumorale.

D'après le brevet européen n° 29 974, on connaît des composés de formule :

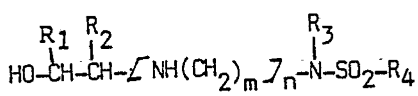

dans laquelle $R_1$ représente un radical alcoyle de 1 à 18 atomes de carbone et $R_2$ représente un atome d'hydrogène ou un radical alcoyle de 1 à 17 atomes de carbone, la somme des atomes de carbone de $R_1$ et $R_2$ allant de 8 à 18, $R_3$ représente un atome d'hydrogène, un radical alcoyle de 1 à 3 atomes de carbone, un radical hydroxyalcoyle de 2 ou 3 atomes de carbone, un radical 2-(diméthylamino)-éthyl ou 3-(diméthylamino)-propyl, $R_4$ représente un radical alcoyle de 1 à 3 atomes de carbone, un radical phényle ou un radical p-tolyle, tandis que m représente un nombre entier de 2 à 6 et n est un nombre entier 0 ou 1.

Ces composés sont présentés comme ayant des propriétés antimicrobiennes.

Les compositions pharmaceutiques selon l'invention contiennent, en association avec un véhicule pharmaceutique, un composé répondant à la formule générale suivante :

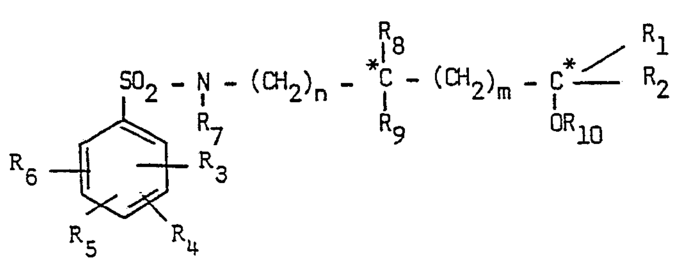

(I)

dans laquelle :

$R_1$, $R_2$ représentent, chacun indépendamment l'un de l'autre :

— un atome d'hydrogène,

— un radical alcoyle inférieur linéaire ou ramifié présentant de 1 à 4 atomes de carbone,

— un radical cycloalcoyle comportant de 3 à 6 atomes de carbone,

— un radical aryle non substitué ou substitué par un groupe nitro, amino, trifluorométhyle, un halogène, ou par un reste alcoyle inférieur linéaire ou ramifié contenant de 1 à 4 atomes de carbone, par un reste alcoxy contenant de 1 à 4 atomes de carbone, notamment méthoxy, par un groupe alcoylsulfonyle —$SO_2R$ dans lequel R est un reste alcoyle contenant de 1 à 4 atomes de carbone, par un groupe sulfamoyle —$SO_2NR'R''$ dans lequel R′ et R″ représentent indépendamment l'un de l'autre un atome hydrogène ou un reste alcoyle contenant de 1 à 4 atomes de carbone ;

$R_3$, $R_4$, $R_5$ et $R_6$ représentent, indépendamment l'un de l'autre :

— un atome d'hydrogène,

— un groupe nitro, amino, trifluorométhyle, un halogène,

— un reste alcoyle inférieur linéaire ou ramifié contenant de 1 à 4 atomes de carbone, un reste alcoxy contenant de 1 à 4 atomes de carbone, un groupe alcoylsulfonyle —$SO_2R$ dans lequel R est un reste alcoyle contenant de 1 à 4 atomes de carbone, un groupe sulfamoyle —$SO_2NR'R''$, dans lequel R′ et R″ représentent, indépendamment l'un de l'autre, un atome d'hydrogène ou un reste alcoyle contenant de 1 à 4 atomes de carbone ;

sous réserve que l'un au moins des substituants $R_3$ à $R_6$ soit différent de l'hydrogène et sous réserve que lorsque l'un des substituants $R_3$ à $R_6$ représente un groupe $CH_3$ en para ou $NH_2$ en para, l'un au moins des autres substituants du noyau aromatique soit différent de l'hydrogène ;

$R_7$, $R_8$ et $R_9$ représentent, indépendamment les uns des autres :

— un atome d'hydrogène,

— un radical alcoyle ayant de 1 à 6 atomes de carbone,

— un radical cycloalcoyle comportant de 3 à 6 atomes de carbone,

— un radical arylalcoyle, notamment phénylalcoyle ou naphtylalcoyle,

dans lequel le radical alcoyle présente de 1 à 4 atomes de carbone, non substitué ou substitué par un groupe nitro, amino, trifluorométhyle, un halogène ou par un reste alcoyle inférieur linéaire ou ramifié contenant de 1 à 4 atomes de carbone, par un reste alcoxy contenant de 1 à 4 atomes de carbone, notamment méthoxy, par un groupe alcoylsulfonyle —$SO_2R$ dans lequel R est un reste alcoyle contenant de 1 à 4 atomes de carbone, par un groupe sulfamoyle —$SO_2NR'R''$ dans lequel R′ et R″ représentent, indépendamment l'un de l'autre, un atome d'hydrogène ou un reste alcoyle contenant de 1 à 4 atomes de carbone ;

$R_{10}$ représente un atome d'hydrogène, un groupe acyle comportant de 1 à 12 atomes de carbone ;

n et m varient, indépendamment l'un de l'autre, de 0 à 10, de préférence de 0 à 5, sous réserve que la somme n + m ne dépasse pas 12, de préférence n'excède pas 5.

Les astérisques apparaissant dans la formule (I) et les formules qui suivent, situent les carbones susceptibles de se présenter sous forme asymétrique.

Afin de faciliter l'appréciation de la nature des différentes classes ou groupes de composés préférés selon l'invention, on les répertoriera dans ce qui suit par groupe G1, G2, etc. Lorsque les formules chimiques pour certaines de ces classes ou certains de ces groupes seront indiquées, elles seront affectées d'un nombre en chiffres romains correspondant respectivement aux indices des groupes G concernés.

L'ensemble des substances, telles que définies par la formule (I) sera donc dans la suite désigné par groupe $G_1$.

Dans la suite du texte, on désignera par médicaments, les compositions pharmaceutiques qui contiennent en association avec un véhicule pharmaceutique, un composé qui répond à la formule générale (I).

Une classe préférée de composés entrant dans les médicaments conformes à l'invention est constituée par ceux du groupe G1 répondant à la formule générale (I) dans laquelle l'un au moins des radicaux $R_3$ à $R_6$ est un radical méthoxy.

Un groupe préféré de composés entrant dans les médicaments selon l'invention répondent à la formule (I) dans laquelle, l'un au moins des radicaux $R_7$, $R_8$ ou $R_9$ représente un radical alcoyle ou cycloalcoyle.

Dans encore un autre groupe préféré de composés entrant dans les médicaments tels que définis ci-dessus, $R_{10}$ représente un radical acétyle, propionyle, phénoxyacétyle, parachlorophénoxyacétyle, pivalyle, adamantyle et embonyle.

Un autre groupe préféré de composés entrant dans les médicaments conformes à l'invention, ci-après dénommé groupe G2, est constitué par ceux définis par la formule (II) suivante :

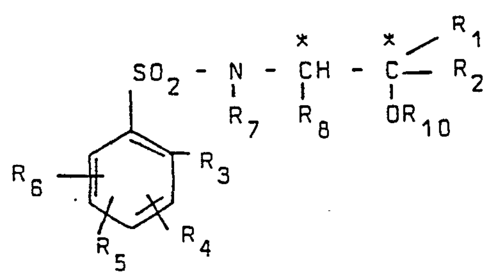

(II)

dans laquelle $R_1$ à $R_8$ ont les significations ci-dessus indiquées, l'un au moins des substituants $R_3$ à $R_6$ étant différent de l'hydrogène.

Ce groupe G2 se distingue essentiellement du groupe G1 en ce que n et m valent simultanément 0 et $R_9$ est un atome d'hydrogène.

Dans le groupe G2, une sous-classe préférée de substances des médicaments selon l'invention, ci-après dénommée G3, est constituée par les composés qui répondent à la formule (II) dans laquelle :

$R_1$ et $R_2$ représentent, indépendamment l'un de l'autre :

— un atome d'hydrogène,

— un radical alcoyle inférieur linéaire ou ramifié présentant de 1 à 4 atomes de carbone,

— un radical cycloalcoyle comportant de 3 à 6 atomes de carbone,

— un radical aryle non substitué ou substitué par un groupe nitro, amino, trifluorométhyle, un halogène ou par un reste alcoyle inférieur linéaire ou ramifié contenant de 1 à 4 atomes de carbone, par un reste alcoxy contenant de 1 à 4 atomes de carbone, notamment méthoxy, par un groupe alcoylsulfonyle —$SO_2R$ dans lequel R est un reste alcoyle contenant de 1 à 4 atomes de carbone, par un groupe sulfamoyle —$SO_2NR'R''$ dans lequel R' et R'' représentent, indépendamment l'un de l'autre, un atome d'hydrogène ou un reste alcoyle contenant de 1 à 4 atomes de carbone ;

$R_3$ représente un radical alcoxy inférieur, linéaire ou ramifié présentant de 1 à 4 atomes de carbone, notamment $OCH_3$ ;

$R_4$, $R_5$ et $R_6$ représentent, indépendamment les uns des autres :

— un atome d'hydrogène,

— un groupe $NO_2$, $NH_2$, $CF_3$ ou un atome d'halogène, notamment le chlore ou le brome,

— un groupe alcoylsulfonyle —$SO_2R$ dans lequel R est un reste alcoyle contenant de 1 à 4 atomes de carbone, un reste alcoxy, notamment $OCH_3$, un groupe sulfamoyle —$SO_2NR'R''$ dans lequel R' et R'' représentent, indépendamment l'un de l'autre, un atome d'hydrogène ou un reste alcoyle contenant de 1 à 4 atomes de carbone ;

$R_7$ et $R_8$ représentent, indépendamment l'un de l'autre :

— un atome d'hydrogène,

— un radical alcoyle ayant de 1 à 6 atomes de carbone,

— un radical cycloalcoyle comportant de 3 à 6 atomes de carbone,

— un radical arylalcoyle dans lequel le radical alcoyle présente de 1 à 4 atomes de carbone et le groupe aryle est de préférence un groupe phényle non substitué ou substitué par un groupe nitro, amino, trifluorométhyle, un halogène ou par un reste alcoyle inférieur linéaire ou ramifié contenant de 1 à 4 atomes de carbone, par un reste alcoxy contenant de 1 à 4 atomes de carbone ;

$R_{10}$ représente un atome d'hydrogène, un groupe acyle, comportant de 1 à 12 atomes de carbone, notamment acétyle, propionyle, phénoxyacétyle, parachlorophénoxyacétyle, pivalyle, adamantyle et embonyle.

Dans le groupe G3, une sous-classe préférée de substances des médicaments conformes à l'invention ci-après dénommée G4, est constituée par les composés de formule (IV) suivante :

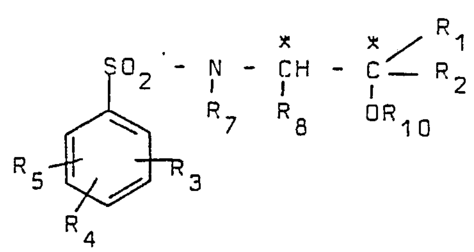

(IV)

dans laquelle $R_1$ à $R_5$, $R_7$, $R_8$ et $R_{10}$ ont les significations indiquées dans la définition du groupe G3, et $R_3$, $R_4$ et $R_5$ sont tous différents de l'hydrogène.

Ce groupe G4 de composés à noyau aromatique trisubstitué se distingue essentiellement du groupe G3 en ce que $R_6$ représente l'hydrogène et $R_3$, $R_4$ et $R_5$ sont tous les trois différents de l'hydrogène.

Dans le groupe G3, une autre sous-classe préférée de substances des médicaments conformes à l'invention, ci-après dénommée G5, est constituée par les composés de formule (V) suivante :

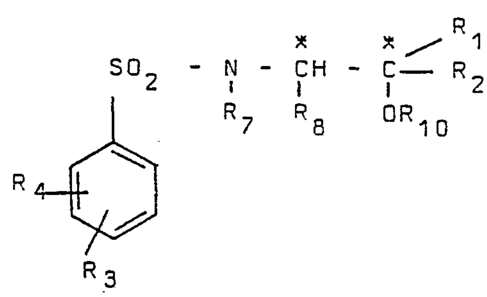

(V)

dans laquelle $R_1$ à $R_4$, $R_7$, $R_8$ et $R_{10}$ ont les significations indiquées dans la définition du groupe G3, et $R_3$ et $R_4$ sont tous les deux différents d'un atome d'hydrogène.

Ce groupe G5 de composés à noyau aromatique disubstitué se distingue essentiellement du groupe G3 en ce que $R_5$ et $R_6$ représentent un atome d'hydrogène, et $R_3$ et $R_4$ sont différents de l'hydrogène.

Dans le groupe G3, une autre sous-classe préférée de substances des médicaments conformes à l'invention, ci-après dénommée G6, est constituée par les composés de formules (VI) suivante :

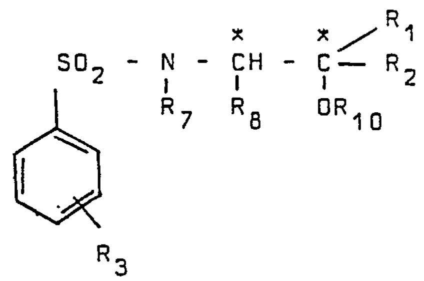

(VI)

dans laquelle $R_1$, $R_2$, $R_3$, $R_7$, $R_8$ et $R_{10}$ ont les significations indiquées dans la définition du groupe G3, et $R_3$ est différent de l'hydrogène.

Ce groupe G6 de composés à noyau aromatique monosubstitué se distingue essentiellement du groupe G3 en ce que $R_4$, $R_5$ et $R_6$ représentent l'hydrogène, et $R_3$ est différent de l'hydrogène.

A l'intérieur des groupes G1, G2, G3, G4, G5 et G6, une classe préférée de substances des médicaments selon l'invention, ci-après dénommée G7, est constituée par les composés de formule (VII) suivante :

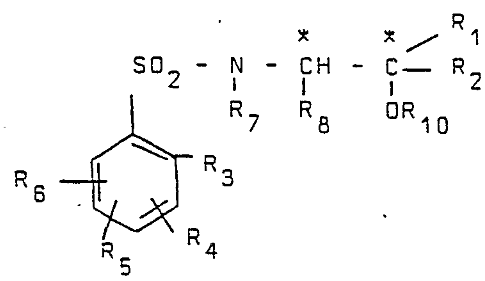

(VII)

Ce groupe G7 se distingue essentiellement des groupes G1 à G6 par le fait que $R_3$ est en position 2 sur le noyau aromatique.

A l'intérieur de ce groupe G7, une classe préférée de substances des médicaments selon l'invention ci-après dénommée G8, est constituée par les composés répondant à la formule (VIII) suivante :

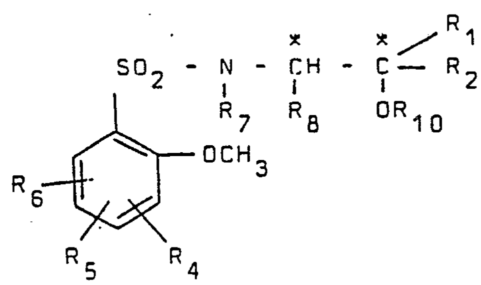

(VIII)

Ce groupe G8 se distingue essentiellement du groupe G7 par le fait que $R_3$ représente un radical méthoxy.

A l'intérieur des groupes G1, G2, G3 et G5, une classe préférée de substances des médicaments conformes à l'invention, ci-après dénommée G9, est constituée par les composés répondant à la formule (IX) suivante :

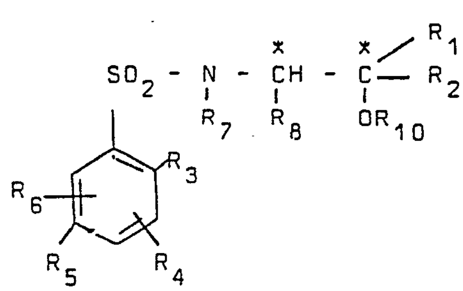

(IX)

Ce groupe G9 se distingue essentiellement des groupes G1, G2, G3 et G5 par le fait que le noyau aromatique comporte au moins deux substituants différents de l'hydrogène qui sont respectivement en position 2 et 5.

A l'intérieur des groupes G1, G2, G3, G4, une classe préférée de substances des médicaments selon l'invention, ci-après dénommée G10, est constituée par les composés répondant à la formule (X) suivante :

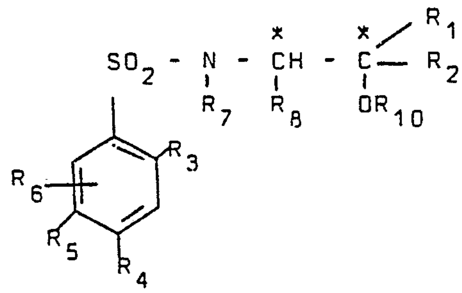

(X)

Ce groupe G10 se distingue essentiellement des groupes G1, G2, G3 et G4 par le fait que le noyau aromatique comporte au moins trois substituants différents de l'hydrogène et qui sont respectivement en position 2, 4, 5 sur le noyau aromatique.

A l'intérieur des groupes G9 et G10, une classe préférée de substances des médicaments selon l'invention, ci-après dénommée G11, est constituée par les composés répondant à la formule (XI) suivante :

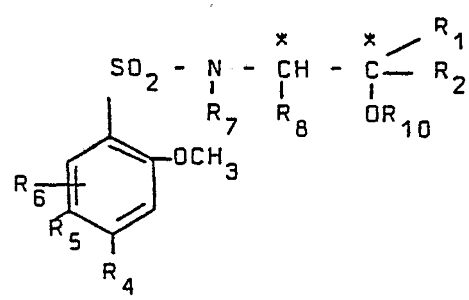

(XI)

Ce groupe G11 se distingue essentiellement des groupes G9 et G10 par le fait que $R_3$ représente un radical méthoxy.

Parmi le groupe G10, une classe préférée de substances des médicaments selon l'invention, ci-après dénommée G12, est constituée par les composés répondant à la formule (XII) suivante :

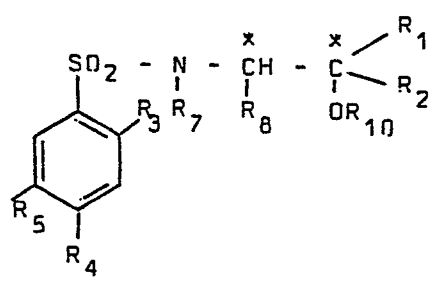

(XII)

Ce groupe G12 se distingue essentiellement du groupe G10 par le fait que $R_6$ représente un atome d'hydrogène.

A l'intérieur du groupe G12, une classe préférée de substances des médicaments selon l'invention, ci-après dénommée G13, est constituée par les composés répondant à la formule (XIII) suivante :

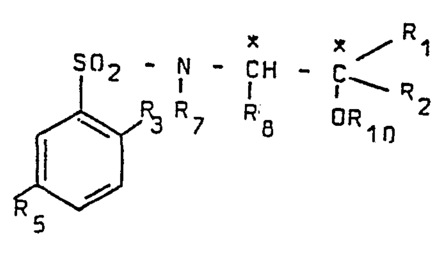

(XIII)

Ce groupe G13 se distingue essentiellement du groupe G12 par le fait que $R_4$ représente un atome d'hydrogène et $R_5$ est différent de l'hydrogène.

A l'intérieur du groupe G12, une autre classe préférée de substances des médicaments selon l'invention, ci-après dénommée G14, est constituée par les composés répondant à la formule (XIV) suivante :

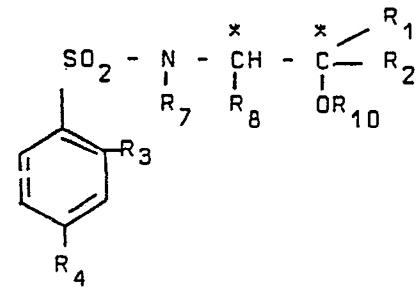

(XIV)

Ce groupe G14 se distingue essentiellement du groupe G12 par le fait que $R_5$ représente un atome d'hydrogène et $R_4$ est différent de l'hydrogène.

A l'intérieur des groupes G1 à G14, une classe préférée de substances des médicaments conformes à l'invention, ci-après dénommée G15, est constituée par les composés répondant à la formule (XV) suivante :

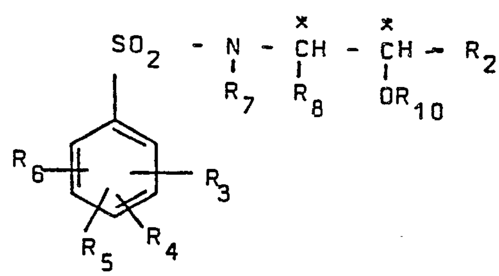

(XV)

Ce groupe G15 se distingue essentiellement des groupes G1 à G14 par le fait que $R_1$ est un atome d'hydrogène.

A l'intérieur des groupes G1 à G15, une classe préférée de substances des médicaments conformes à l'invention, ci-après dénommée G16, est constituée par les composés répondant à la formule (XVI) suivante :

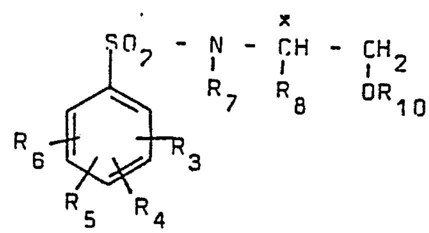

(XVI)

Ce groupe G16 se distingue essentiellement des groupes G1 à G15 par le fait que $R_1$ et $R_2$ représentent simultanément un atome d'hydrogène.

A l'intérieur des groupes G12 à G16, une classe préférée de substances des médicaments conformes à l'invention, ci-après dénommée G17, est constituée par les composés répondant à la formule (XVII) suivante :

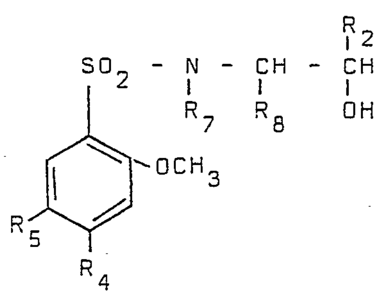

(XVII)

Ce groupe G17 se distingue essentiellement des groupes G12 à G16 par le fait que :

$R_1$ représente l'hydrogène,

$R_2$ représente H ou $CH_3$,

$R_3$ représente $OCH_3$,

$R_4$ et $R_5$, indépendamment l'un de l'autre, sont choisis dans le groupe comprenant : H, $NO_2$, $NH_2$, Cl, Br, $OCH_3$, $SO_2C_2H_5$, $SO_{2i}C_3H_7$, $SO_{2n}C_3H_7$, de préférence l'un au moins des radicaux étant différent de l'hydrogène,

$R_7$ représente l'hydrogène ou $CH_3$,

$R_8$ représente H ou $C_2H_5$,

$R_{10}$ représente l'hydrogène.

A l'intérieur du groupe G17, une classe préférée de substances des médicaments selon l'invention, ci-après dénommée G18, est constituée par les composés répondant à la formule (XVII) dans laquelle $R_2$ représente un radical $CH_3$, $R_7$ et $R_8$ représentent un atome d'hydrogène.

A l'intérieur du groupe G17, une autre classe préférée de substances des médicaments selon l'invention, ci-après dénommée G19, est constituée par les composés répondant à la formule (XVII) dans laquelle $R_2$ et $R_7$ représentent un atome d'hydrogène et $R_8$ représente un groupe $C_2H_5$.

A l'intérieur des groupes G18 et G19, une classe préférée de substances des médicaments selon l'invention, ci-après dénommée G20, est constituée par les composés répondant à la formule (XVII) dans laquelle $R_4$ et $R_5$ sont choisis dans le groupe comprenant H, Cl, $NO_2$, $NH_2$, Br.

A l'intérieur des groupes G18, G19, G20, une classe préférée de substances des médicaments conformes à l'invention, ci-après dénommée G21, est constituée par les composés dans lesquels $R_5$ est en position 5 et est choisi parmi Br, Cl, $NO_2$, $NH_2$.

Parmi le groupe G17, une classe préférée de substances des médicaments conformes à l'invention, ci-après dénommée G22, est constituée par les composés dans lesquels $R_2$ représente un atome d'hydrogène, $R_7$ représente $CH_3$, $R_8$ représente un atome d'hydrogène.

A l'intérieur du groupe G17, une autre classe préférée de substances des médicaments conformes à l'invention, ci-après dénommée G23, est constituée par les composés dans lesquels $R_2$ représente $CH_3$, $R_7$ représente un atome d'hydrogène et $R_8$ représente $C_2H_5$.

A l'intérieur des groupes G22 et G23, une classe préférée de substances des médicaments conformes à l'invention, ci-après dénommée G24, est constituée par les composés dans lesquels $R_5$ en position 5 représente Br, Cl, $NO_2$.

A l'intérieur du groupe G18, une classe préférée de substances des médicaments conformes à l'invention, ci-après dénommée G25, est constituée par les composés dans lesquels $R_4$ en position 4 représente $NO_2$, $NH_2$, H.

A l'intérieur des groupes G18, G19, G20, G21, G22, une classe préférée de substances des médicaments conformes à l'invention, ci-après dénommée G26, est constituée par les composés dans lesquels $R_5$ en position 5, représente H, Cl, Br, $NO_2$.

A l'intérieur des groupes G18 et G19, une classe préférée de substances des médicaments conformes à l'invention, ci-après dénommée G27, est constituée par les composés dans lesquels $R_4$ en position 4, représente $NO_2$ ou $NH_2$.

A l'intérieur des groupes G18 et G19, une classe préférée de substances des médicaments conformes à l'invention, ci-après dénommée G28, est constituée par les composés dans lesquels $R_5$ en position 5, représente $NH_2$, $NO_2$, Cl.

L'invention concerne également les isomères optiques et les diastéréoisomères des composés de formule (I) ainsi que les sels physiologiquement acceptables de ces isomères optiques.

Les composés particulièrement préférés selon l'invention sont ceux de formule :

14

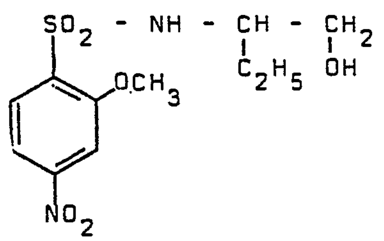

Composé n° 312

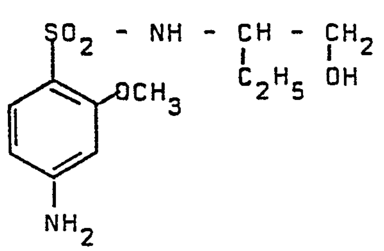

313

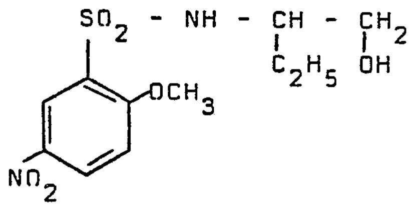

314

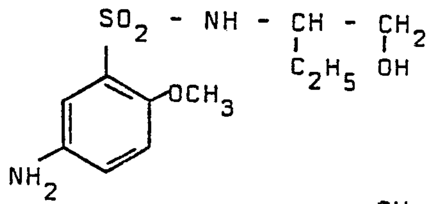

315

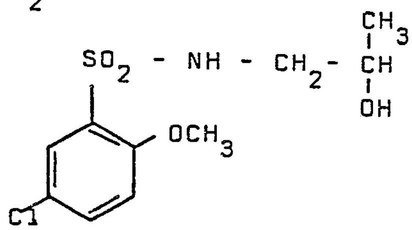

318

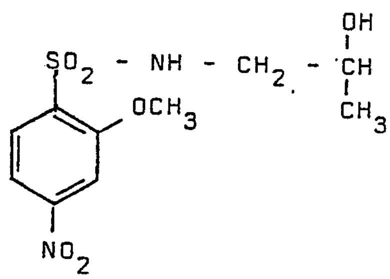

319

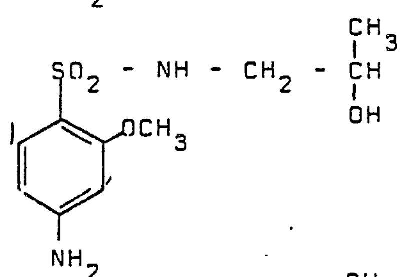

320

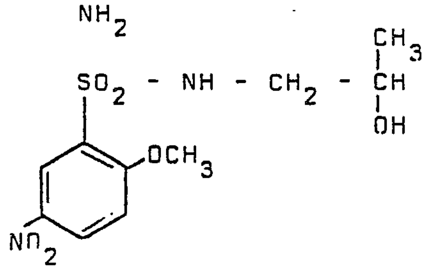

321

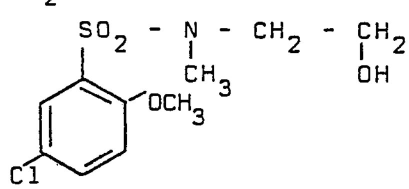

324

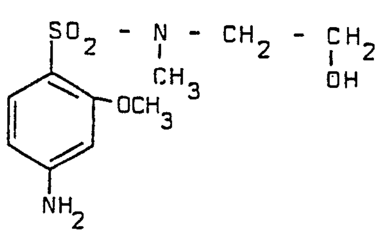

326

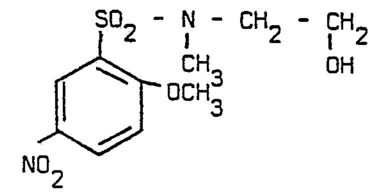

327

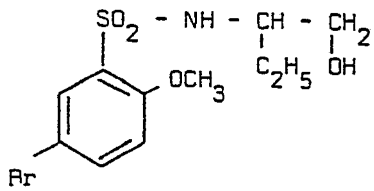

645

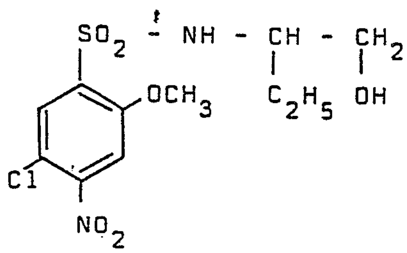

316

16

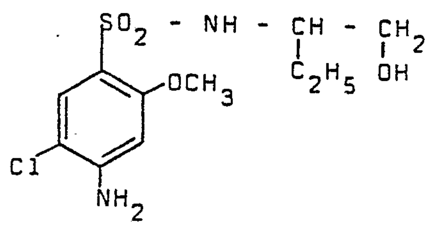

317

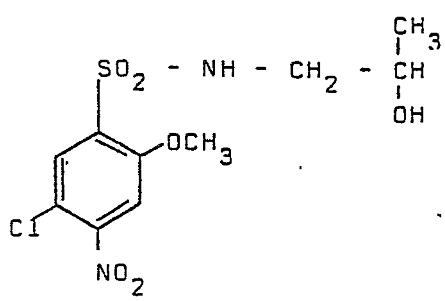

322

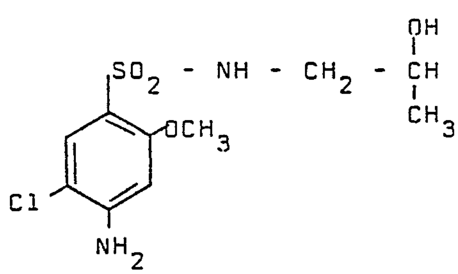

323

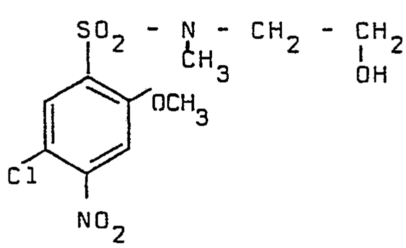

329

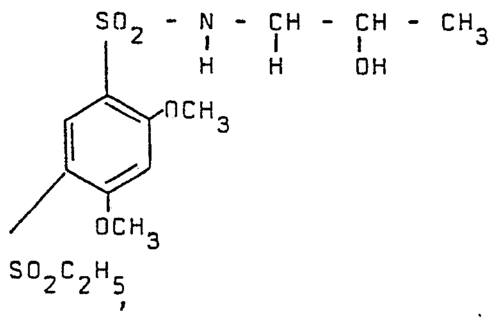

1 074

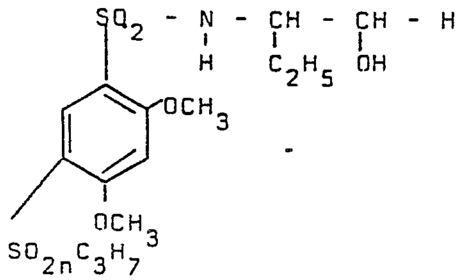

1 164

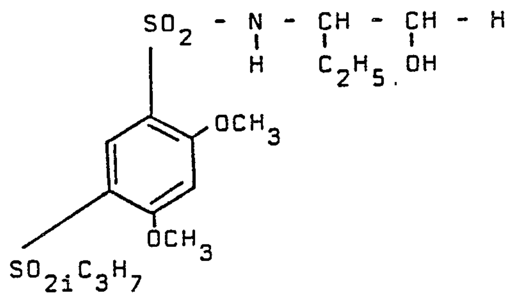

1 167

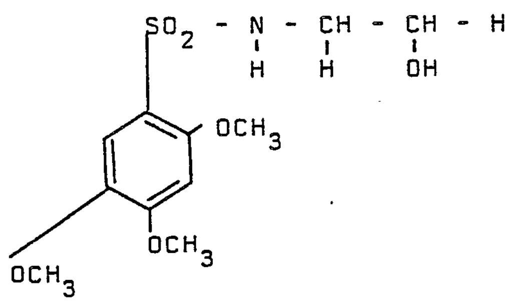

1 152

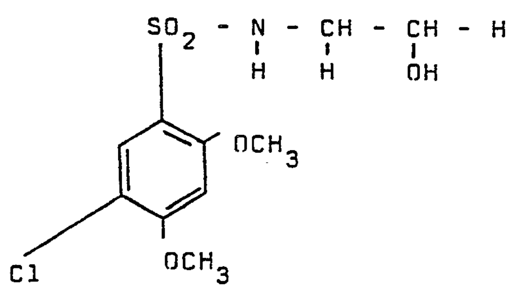

1 153

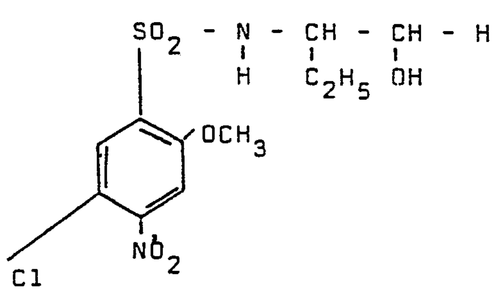

1 195

Pour former les médicaments selon l'invention, on peut avoir recours aux sulfohalogénures, notamment aux sulfochlorures de formule :

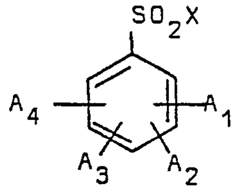

(XVII)

dans laquelle :

X est un groupe halogène, notamment brome ou de préférence chlore ;

$A_1$, $A_2$, $A_3$, $A_4$ représentent, indépendamment les uns des autres :

— un atome d'hydrogène,

— un groupe nitro, trifluorométhyle, un halogène,

— un radical alcoyle inférieur linéaire ou ramifié, présentant de 1 à 4 atomes de carbone,

— un radical alcoxy ayant de 1 à 4 atomes de carbone, notamment méthoxy,

— un radical alcoylsulfonyle —$SO_2R$, dans lequel R est un reste alcoyle pouvant contenir jusqu'à 4 atomes de carbone,

— un radical sulfamoyle —$SO_2NR'R''$, dans lequel R' et R" représentent, indépendamment l'un de l'autre, un atome d'hydrogène ou un reste alcoyle pouvant contenir jusqu'à 4 atomes de carbone ; sous réserve que l'un au moins des substituants $A_1$ à $A_4$ soit différent de l'hydrogène et sous réserve que lorsque $A_1$ à $A_4$ représentent un groupe $CH_3$ en para ou $NH_2$ en para, l'un au moins des autres substituants soit différent de l'hydrogène.

Les sulfochlorures de formule ci-dessous indiquée :

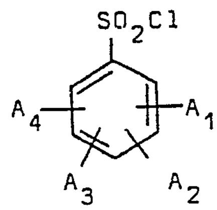

(XVIII)

dans laquelle $A_1$, $A_2$, $A_3$ et $A_4$ ont les significations ci-dessus indiquées, sont de façon générale disponibles dans le commerce.

Lorsqu'ils ne sont pas disponibles dans le commerce, on peut les synthétiser soit par des méthodes classiques, soit pour certains d'entre eux par les méthodes exposées ci-après. Il s'agit notamment des sulfohalogénures de formule :

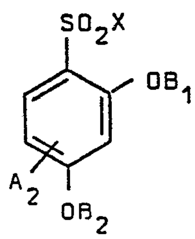

(XIX)

dans laquelle :

X est un halogène,

$B_1$ représente un radical alcoyle présentant de 1 à 4 atomes de carbone, de préférence méthyle,

$B_2$ représente un radical alcoyle présentant de 1 à 4 atomes de carbone, de préférence méthyle,

$A_3$, de préférence en position 5, représente :

— un atome d'hydrogène,

— un groupe nitro, trifluorométhyle, un halogène, notamment le chlore ou le brome,

— un radical alcoyle inférieur linéaire ou ramifié, présentant de 1 à 4 atomes de carbone,

— un radical alcoxy ayant de 1 à 4 atomes de carbone, notamment méthoxy,

un radical alcoylsulfonyle —$SO_2R$, dans lequel R est un reste alcoyle pouvant contenir jusqu'à 4 atomes de carbone, notamment $SO_2CH_3$, $SO_2C_2H_5$, $SO_{2n}C_3H_7$, $SO_{2i}C_3H_7$,

— un radical sulfamoyle —$SO_2NR'R''$, dans lequel R' et R" peuvent représenter indépendamment l'un de l'autre, un atome d'hydrogène ou un reste alcoyle pouvant contenir jusqu'à 4 atomes de carbone.

Les méthodes dont question ci-après, se rapportent à la fabrication des sulfohalogénures de formule (XX) :

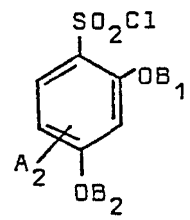

(XX)

qui se différencie de la formule (XIX) par le fait que X représente le chlore, étant entendu que ces méthodes sont directement transposables aux autres sulfohalogénures.

Une première desdites méthodes utilise comme matière première une arylamine de formule :

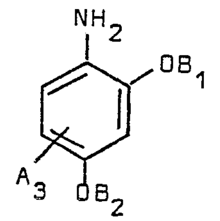

(XXI)

dans laquelle $R_1$, $B_2$ et $A_3$ ont les significations ci-dessus indiquées, et à partir de laquelle :

a) on forme le sel de diazonium notamment le chlorure de diazonium de formule (XXII). Ce sel de diazonium est notamment obtenu en faisant réagir l'arylamine au sein d'une solution de l'acide halohydrique correspondant, notamment d'acide chlorhydrique, avec une solution de nitrite d'un métal alcalin, et en maintenant le mélange réactionnel à une température de préférence inférieure à environ 10 °C ;

b) on fait ensuite réagir le sel de diazonium obtenu en solution avec de l'anhydride sulfureux. On opère de préférence en présence d'acide acétique ainsi que d'un catalyseur apte à contribuer à la transformation du groupe diazonium en groupe sulfohalogénure, notamment sulfochlorure. Ce catalyseur est, par exemple, à base de cuivre (réaction de Sandmeyer modifiée).

Les étapes a) et b) de cette réaction appliquée à titre d'exemple à la préparation des sulfochlorures de formule (XX) dans laquelle X représente le chlore, peuvent être représentées comme suit :

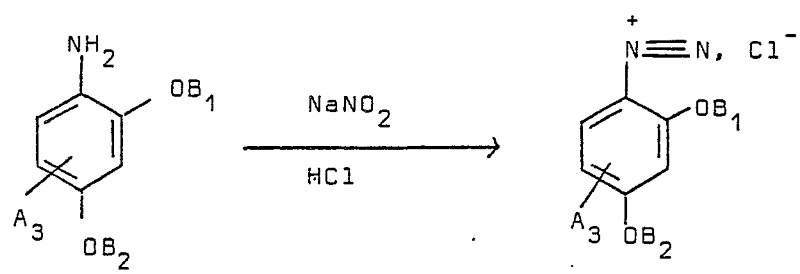


(XXII)

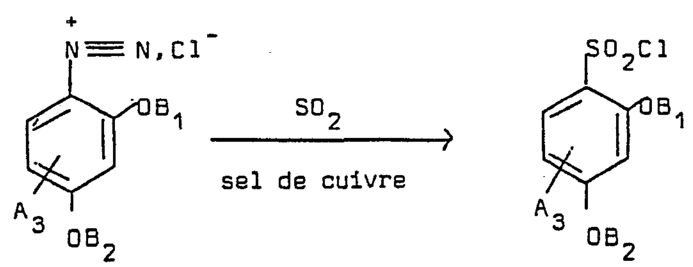


(XX)

A titre d'arylamines préférées pour la préparation des sulfochlorures de formule (XX) on peut citer :

la diméthoxy-2,4 aniline,
la chloro-5 diméthoxy-2,4 aniline,
la bromo-5 diméthoxy-2,4 aniline,
la triméthoxy-2,4,5 aniline,
la diméthoxy-2,4 méthylsulfonyl-5 aniline,
la diméthoxy-2,4 éthylsulfonyl-5 aniline,
la diméthoxy-2,4 propylsulfonyl-5 aniline,
la diméthoxy-2,4 isopropylsulfonyl-5 aniline,
la chloro-5 méthoxy-2 nitro-4 aniline.

Une autre méthode pour préparer les sulfohalogénures de formule (XIX), notamment les sulfochlorures de formule (XX) dans lesquels $B_1$, $B_2$ et $A_3$ ont les significations ci-dessus indiquées, peut consister à procéder par sulfonation ou halogénosulfonation de préférence chlorosulfonation du composé de formule (XXIII) dans laquelle $B_1$, $B_2$, $A_3$ ont les significations sus-indiquées :

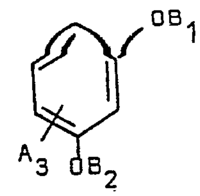

(XXIII)

Dans le cas de la sulfonation (par réaction du composé de formule (XXIII) avec de l'acide sulfurique) on obtient dans une première étape, l'acide sulfonique de formule (XXIV) :

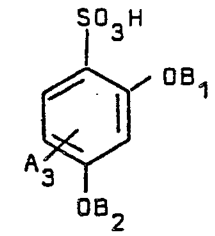

(XXIV)

L'acide de formule (XXIV) obtenu peut ensuite être transformé en sel d'une base organique ou minérale, par action de la base appropriée telle que l'hydroxyde de sodium, l'hydroxyde de potassium ou la pyridine.

Ce sel est représenté par la formule (XXV) suivante :

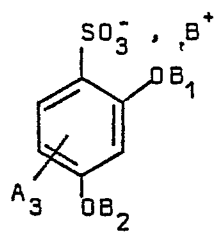

(XXV)

dans laquelle $B^+$ représente un métal, notamment alcalin ou alcalino-terreux, et $A_3$, $B_1$ et $B_2$ ont l'une quelconque des significations indiquées ci-dessus. Le sulfohalogénure de formule (XIX), notamment le chlorure de formule (XX) est alors obtenu par action sur le composé de formule (XXIV) — ou sur un sel organique ou minéral correspondant de formule (XXV) — d'un agent chlorant, tel que le chlorure de thionyle, le pentachlorure de phosphore, ou l'oxychlorure de phosphore.

A titre d'exemple, le schéma réactionnel de l'obtention des composés de formule (XX) dans laquelle $A_3$, $B_1$ et $B_2$ ont l'une quelconque des significations indiquées ci-dessus à partir des composés de formule (XXIII) peut se représenter comme suit :

(XXIII)

(XXIV)

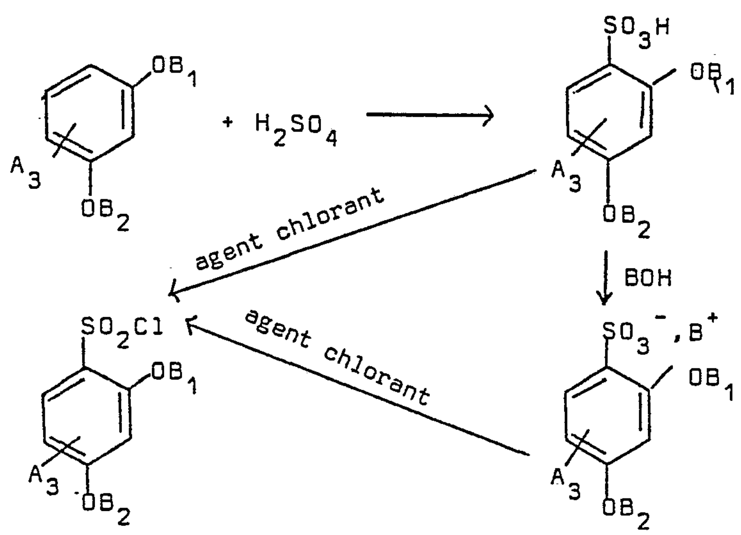


(XX)

(XXV)

Dans le cas de la chlorosulfonation, on fait réagir l'acide chlorosulfonique sur le composé de formule (XXIII) dans laquelle $B_1$, $B_2$ et $A_3$ ont les significations ci-dessus indiquées.

La réaction peut s'écrire :

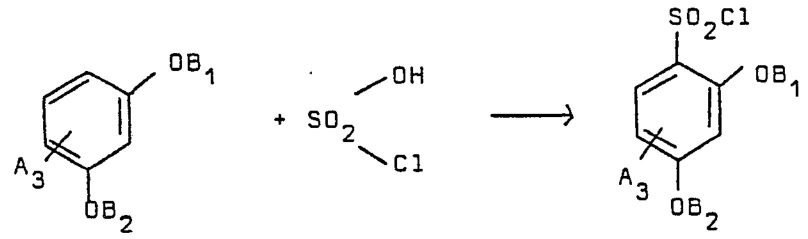

Les composés de formule (I) peuvent être obtenus par réaction d'une amine de formule :

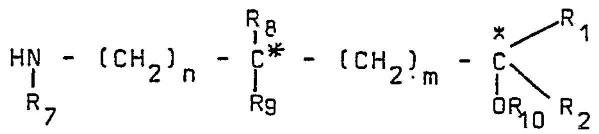
(XXVI)

dans laquelle :

n et m ont les signifcations indiquées ci-dessus ;

$R_1$, $R_2$, $R_7$, $R_8$, $R_9$ et $R_{10}$ ont les significations ci-dessus indiquées ;

sur un sulfohalogénure de formule :

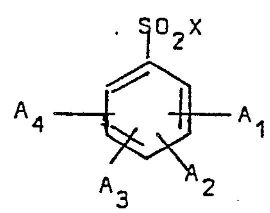
(XVII)

dans laquelle X est le chlore ou le brome, $A_1$, $A_2$, $A_3$ et $A_4$ ont l'une quelconque des significations données pour $R_3$, $R_4$, $R_5$ et $R_6$, à l'exception de $NH_2$.

Par réduction par hydrogénation catalytique ou par réduction chimique — des composés de formule (I) dans lesquels l'un au moins des groupes $R_3$ à $R_6$ représente $NO_2$, on obtient les composés de formule (I) dans lesquels le ou les groupes $R_3$ à $R_6$ correspondants sont $NH_2$.

Un groupe d'amino-alcools préférés utilisés dans la préparation des composés de formule (I) est constitué par les suivants :

éthanolamine,
hydroxy-2 propylamine,
amino-2 butanol,
amino-2 hexanol-1,
amino-2 méthyl-3 butanol-1
amino-2 méthyl-3 butanol,
amino-5 pentanol,
amino-6 méthyl-2 heptanol-2,
amino-4 butanol-1,
amino-2 méthyl-4 pentanol-1,
amino-8 octanol-1.

Les exemples suivants relatifs à la préparation d'un certain nombre de nouveaux benzènesulfonamidoalcools, servent à illustrer l'invention, mais ne sont pas limitatifs. On décrira d'abord des exemples de fabrication des matières premières à partir desquelles ont ensuite été obtenues les substances décrites dans les exemples.

Préparation du chlorure de triméthoxy-2,4,5 benzène sulfonyle.

Elle peut être effectuée selon deux procédés

1^er^ procédé

Dans un tricol de 500 $cm^3$, muni d'un thermomètre, d'une ampoule à réactif, d'une garde à chlorure de calcium et d'un système d'agitation magnétique, sont placés 42 g (0,25 mole) de triméthoxy-1,2,4 benzène en solution dans 200 $cm^3$ de chloroforme pur. Au milieu réactionnel, placé sous atmosphère

d'azote et refroidi vers − 10 °C, sont ajoutés goutte à goutte 80 $cm^3$ d'acide chlorosulfonique. Il se forme successivement un précipité laiteux blanc-crème, puis une solution verdâtre qui vire au brun. L'addition terminée, le milieu réactionnel est laissé en contact 1 h à température ambiante, puis est versé sur de la glace pilée. Le précipité obtenu est extrait par du chloroforme ; la phase organique est ensuite séchée sur sulfate de sodium puis évaporée sous pression réduite. Le résidu brun formé est lavé avec le minimum de toluène jusqu'à l'obtention d'un solide beige qui est ensuite chromatographié sur colonne de silice.

L'élution avec du benzène, puis avec un mélange benzène — chloroforme (50-50) donne le chlorure de triméthoxy-2,4,5 benzènesulfonyle.

Rdt 46 % F 147 °C

RMN ($CDCl_3$) à 80 MHz :

δ 7,29 ppm (s 1H ArH) ; δ 6,55 ppm (s 1H ArH) ; δ 3,93, 3,78 et 3,53 ppm (3s 9H $OCH_3$)

IR (KBr)$\gamma SO_2$, as 1 350 $cm^{-1}$, s 1 160 $cm^{-1}$.

## 2e procédé

Il comporte 4 étapes :

Diméthoxy-3,4 chlorobenzène :

Dans un tricol d'un litre, muni d'un système d'agitation magnétique, d'un thermomètre, d'une garde à chlorure de calcium et d'une ampoule à réactif, sont introduits à froid vers 0 °C et successivement 138 g (1 mole) de vératrole puis goutte à goutte 135 g (1 mole) de chlorure de sulfuryle. L'addition terminée, le milieu réactionnel est ramené à température ambiante, puis après contact une heure est distillé sous pression réduite.

Rdt 83 % Pt : 120 °C

sous 1 999,03 Pa (15 mm Hg)

Diméthoxy-4,5 nitro-2 chlorobenzène :

Dans un tricol d'un litre, muni d'un système d'agitation magnétique, d'un thermomètre et d'une ampoule à réactif, sont introduits successivement 143,9 g (0,83 mole) de diméthoxy-3,4 chlorobenzène, puis goutte à goutte 166 $cm^3$ d'acide nitrique (d = 1,38), sans que la température dépasse 25 °C. L'addition terminée, le mélange réactionnel est laissé 1,5 h en contact puis filtré.

Rdt 95 % F 105 °C

Triméthoxy-2,4,5 nitrobenzène :

Dans un ballon de 2 litres, muni d'un réfrigérant, sont introduits successivement une solution de potasse méthanolique (100 g de KOH dans 500 $cm^3$ de méthanol), puis 100 g (0,46 mole) de diméthoxy-4,5 nitro-2 chlorobenzène et du carborandum. Le mélange est porté à l'ébullition au reflux 6 h. Après refroidissement, le milieu réactionnel est filtré ; le précipité obtenu est lavé avec du méthanol.

Rdt 95 % F 129 °C

Triméthoxy-2,4,5 aniline :

Dans un ballon de 500 $cm^3$, muni d'un réfrigérant, sont ajoutés successivement 21,3 g (0,1 mole) de triméthoxy-2,4,5 nitrobenzène, 80 g de chlorure stanneux chimiquement pur pour miroitiers, 100 $cm^3$ d'une solution d'acide chlorhydrique (d = 1,18) et du carborandum. Le mélange est porté à l'ébullition à reflux 1 h. Après refroidissement, une solution de lessive de soude est ajoutée jusqu'à dissolution du précipité. La solution obtenue est extraite par du chlorure de méthylène. Les extraits organiques sont séchés sur sulfate de sodium puis évaporés sous pression réduite. Le résidu obtenu est cristallisé dans de l'éthanol.

Rdt 80 % F 94 °C

Chlorure de triméthoxy-2,4,5 benzènesulfonyle :

Dans un tricol de 250 $cm^3$, muni d'un système d'agitation et d'un thermomètre, sont introduits successivement 18,3 g (0,1 mole de triméthoxy-2,4,5 aniline puis 50 $cm^3$ d'une solution d'acide chlorhydrique (d = 1,18). Après un contact de 4 heures, l'amine est diazotée à − 5 °C par addition d'une solution de nitrite de sodium (10 g de $NaNO_2$ dans 50 $cm^3$ d'eau). Le sel de diazonium obtenu est versé lentement dans un tricol, chauffé à 40 °C et sous atmosphère d'azote, contenant 200 $cm^3$ d'acide acétique saturé en anhydride sulfureux et 7 g de chlorure cuivrique. Le mélange est porté 2 h à 60 °C, puis est versé sur de la glace pilée.

Rdt 35 % F 147 °C

## Préparation du chlorure de chloro-5 diméthoxy-2,4 benzène-sulfonyle

Elle est effectuée en 2 étapes à partir du diméthoxy-1,3 benzène :

1ère étape : Obtention du diméthoxy-2,4 chlorobenzène

Elle s'effectue en suivant la technique de G. Castelfranchi et G. Borra rapportée dans Annali di Chimica, 1953, *43*, 293.

Dans un tricol de 500 $cm^3$, muni d'un système d'agitation magnétique, d'un thermomètre, d'une

garde à chlorure de calcium et d'une ampoule à réactif, et refroidi vers 10 °C, sont introduits successivement 97,5 g (0,70 mole) de chlorure de sulfuryle. Une fois l'addition terminée, la solution est ramenée à la température ambiante et laissée en contact 2 h puis distillée.

Rdt 85 % PE : 137 °C sous 2 399,80 Pa (18 mm Hg)

2[e] étape : Passage au chlorure de chloro-5 diméthoxy-2,4 benzènesulfonyle

Dans un tricol de 500 $cm^3$, muni d'un système d'agitation magnétique, d'une garde à chlorure de calcium, d'un thermomètre et d'une ampoule à réactif, sont introduits 35 g (0,2 mole) de diméthoxy-2,4 chlorobenzène en solution dans 250 $cm^3$ de chloroforme pur. La solution est refroidie vers 0 °C, puis est additionnée goutte à goutte de 50 $cm^3$ (0,75 mole) d'acide chlorosulfonique. Une fois l'addition terminée, le milieu réactionnel est ramené à température ambiante, puis laissé en contact 3 h ; il est ensuite versé sur de la glace pilée. Le mélange obtenu est épuisé au chloroforme. Les extraits organiques sont séchés sur sulfate de sodium puis concentrés jusqu'à cristallisation. Le solide obtenu est recristallisé dans un mélange éther éthylique/benzène.

Rdt 74 % F 175 °C

RMN ($CDCl_3$) à 80 MHz :

δ 7,87 ppm (s 1H ArH) ; δ 6,55 ppm (s 1H ArH) ;

δ 4 et 3,96 ppm (2s 6H $OCH_3$)

IR (KBr) : $\gamma SO_2$, as 1 385 $cm^{-1}$, s 1 170 $cm^{-1}$

Préparation du chlorure de bromo-5 diméthoxy-2,4 benzènesulfonyle

Elle est effectuée en 2 étapes à partir du diméthoxy-1,3 benzène

1ère étape : Obtention du diméthoxy-2,4 bromobenzène

Elle s'effectue en suivant le protocole de S.T. FENG et K.Y. CHIU rapportée dans Hsûeh Pao, 1959, *25*, 277.

Dans un tricol de 250 $cm^3$, muni d'un système d'agitation magnétique, d'un thermomètre, et refroidi vers 10 °C, sont ajoutés successivement 27,6 g (0,2 mole) de diméthoxy-1,3 benzène puis par petites portions 36 g (0,2 mole) de N-bromosuccinimide. Une fois l'addition terminée, le milieu réactionnel est ramené à température ambiante et laissé en contact 2 h. Après lavage à l'eau et extraction au chloroforme, les extraits organiques sont séchés sur sulfate de sodium puis concentrés sous pression réduite. Le liquide résiduel est distillé.

Rdt 82 % PE : 150 °C sous 1 999,83 Pa (15 mm Hg)

2[e] étape : Passage au chlorure de bromo-5 diméthoxy-2,4 benzènesuflonyle

Il est obtenu selon le même protocole que celui décrit dans l'exemple 3 pour préparer le chlorure de chloro-5 diméthoxy-2,4 benzènesulfonyle. En utilisant 65,1 g (0,3 mole) de diméthoxy-2,4 bromobenzène et 100 $cm^3$ d'acide chlorosulfonique, le rendement est de 77 %.

F 195 °C

RMN ($CDCl_3$) à 60 MHz

δ 7,85 ppm (s 1H ArH) ; δ 6,70 ppm (s 1H ArH) ;

δ 3,92 et 3,88 ppm (2s 6H $OCH_3$) ;

IR (KBr) ; $\gamma SO_2$, as 1 385 $cm^{-1}$, s 1 165 $cm^{-1}$

Chlorures d'alkylsulfonyl-5 diméthoxy-2,4 benzènesulfonyles

Ils sont obtenus à partir du chlorure de diméthoxy-2,4 benzènesulfonyle dont la préparation est rapportée par C. M. Suter et H. L. Hansen dans J. of Am. Chem. Soc. 1933, *55*, 2 080. Le schéma général de leur préparation est le suivant :

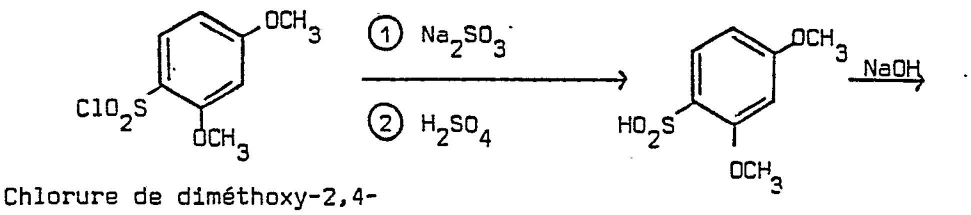


Chlorure de diméthoxy-2,4-benzènesulfonyle

Acide diméthoxy-2,4 benzènesulfinique

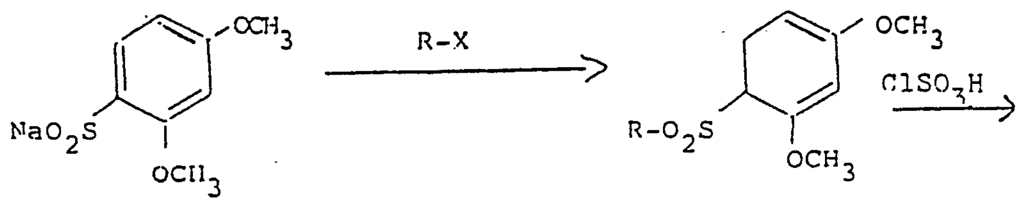


Diméthoxy-2,4 benzène-sulfinate de sodium

(Diméthoxy-2,4 phényl) alkylsulfone

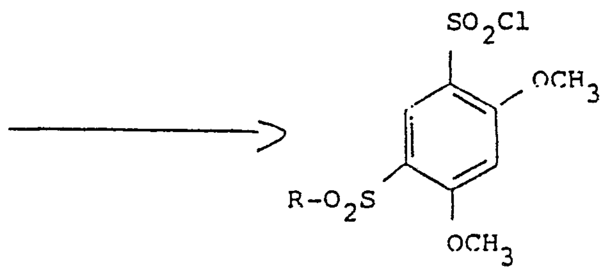

Chlorure d'alkylsulfonyl-5 diméthoxy-2,4 benzènesulfonyle

Acide diméthoxy-2,4 benzènesulfinique :

A une solution aqueuse contenant 129 g (0,974 mole) de sulfite de sodium sont ajoutés par petites portions et sous agitation 115 g (0,487 mole) de chlorure de diméthoxy-2,4 benzènesulfonyle. Durant cette opération, le pH est maintenu alcalin par addition de lessive de soude. Après 3 h de contact, le milieu réactionnel est filtré ; le filtrat est acidifié par de l'acide sulfurique 2N jusqu'à précipitation de l'acide sulfinique.

Rdt 72 % F 122 °C

Diméthoxy-2,4 benzènesulfinate de sodium

Il est obtenu par addition de la quantité stœchiométrique d'hydroxyde de sodium dans l'eau. La solution de sulfinate est évaporée à sec.

(Diméthoxy-2,4 phényl) alkylsulfones :

Protocole général :

Dans un ballon de 500 cm³, muni d'un réfrigérant et d'un système d'agitation magnétique, sont ajoutés successivement 0,1 mole de diméthoxy-2,4 benzènesulfinate de sodium, 250 cm³ d'isopropanol, puis 0,15 mole d'halogénure d'alkyle, de préférence un iodure. Le mélange est porté à l'ébullition à reflux 5 à 30 h selon l'halogénure mis en œuvre. Après refroidissement, le milieu réactionnel est évaporé à sec. Le résidu est repris par de l'eau puis est extrait par du chloroforme. L'évaporation de la phase organique, après séchage sur sulfate de sodium fournit une huile qui est cristallisée dans du benzène.

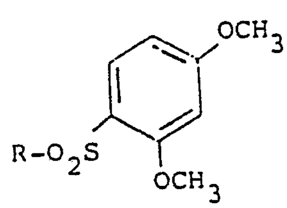

| R | PM | Temps de chauffage | F°C | Rdt % |
|---|---|---|---|---|
| $CH_3$ | 216 | 5 h | 109 | 65 |

(Suite)

| R | PM | Temps de chauffage | F°C | Rdt % |
|---|---|---|---|---|
| $C_2H_5$ | 230 | 7 h | 94 | 70 |
| $nC_3H_7$ | 244 | 15 h | 70 | 90 |
| $iC_3H_7$ | 244 | 30 h | ! 100 | 64 |

Chlorures d'alkylsulfonyl-5 diméthoxy-2,4 benzènesulfonyles :

Protocole général :

Dans un tricol d'un litre, muni d'un système d'agitation magnétique, d'une garde à chlorure de calcium, d'un thermomètre et d'une ampoule à réactif, est introduite une solution de (diméthoxy-2,4 phényl) alkylsulfone (0,2 mole) dans 200 $cm^3$ de chloroforme pur. Après refroidissement vers − 10 °C, sont ajoutés goutte à goutte 100 $cm^3$ d'acide chlorosulfonique. Une fois l'addition terminée, le mélange est laissé en contact 0,5 h à − 10 °C, puis est ramené à température ambiante et laissé sous agitation pendant 7 h ; il est ensuite versé sur de la glace pilée. Le mélange obtenu est extrait par du chloroforme. La phase organique est séchée sur sulfate de sodium, filtrée, puis évaporée sous pression réduite. Le résidu est cristallisé dans du benzène.

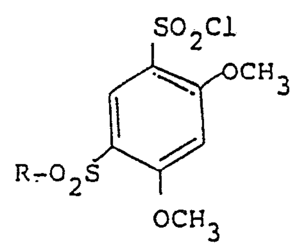

| R | PM | F°C | Rdt % |
|---|---|---|---|
| $CH_3$ | 314,5 | 204 | 52 |
| $C_2H_5$ | 328,5 | 191 | 77 |
| $nC_3H_7$ | 342,5 | 169 | 87 |
| $iC_3H_7$ | 342,5 | 218 | 66 |

Chlorure de diméthoxy-2,4 méthylsulfonyl-5 benzènesulfonyle

RMN (DMSO) à 80 MHz :
δ 8,2 ppm (s 1H ArH) ; δ 6,9 ppm (s 1H ArH) ; δ 4,1 ppm et 4,05 ppm (2s 6H $OCH_3$) ; 3,2 ppm (s 3H $CH_3$).
IR (KBr) :
ν $SO_2$-Cl, as 1 360 $cm^{-1}$, s 1 170 $cm^{-1}$ ;
ν $SO_2$-$CH_3$, as 1 300 $cm^{-1}$, s 1 140 $cm^{-1}$.

Chlorure d'éthylsulfonyl-5 diméthoxy-2,4 benzènesulfonyle

RMN (DMSO) à 80 MHz :

δ 8,2 ppm (s 1H ArH) ; δ 6,93 ppm (s 1H ArH) ; δ 4,15 ppm et 4,1 ppm (2s 6H $OCH_3$) ; δ 3,25 ppm (q 2H-$CH_2$-) ; δ 1,18 ppm (t 3H $CH_3$-).
IR (KBr) :
γ $SO_2$-Cl, as 1 370 $cm^{-1}$, s 1 175 $cm^{-1}$ ;
γ $SO_2$-$CH_2$-, as 1 315 $cm^{-1}$, s 1 140 $cm^{-1}$.

Chlorure de diméthoxy-2,4 propylsulfonyl-5 benzènesulfonyle

RMN (DMSO) à 80 MHz :
δ 8,05 ppm (s 1H ArH) ; δ 6,72 ppm (s 1H ArH) ; 3,92 et 3,87 ppm (2s 6H $OCH_3$) ; δ 3,2 ppm (t 2H-$CH_2$-$SO_2$-) ; δ 1,45 ppm (m 2H-$CH_2$-) ; δ 0,85 ppm (t 3H $CH_3$).
IR (KBr) :
γ $SO_2Cl$, as 1 370 $cm^{-1}$, s 1 180 $cm^{-1}$ ;
γ $SO_2$-$CH_2$, as 1 315 $cm^{-1}$, s 1 135 $cm^{-1}$.

Chlorure d'isopropylsulfonyl-5 diméthoxy-2,4 benzènesulfonyle

RMN (DMSO) à 80 MHz :
δ 8,02 ppm (s 1H ArH) ; δ 6,7 ppm (s 1H ArH) ; δ 3,92 et 3,85 ppm (2s 6H $OCH_3$) ; δ 3,48 ppm (m 1H-CH-) ; δ 1,11 et 1,03 ppm (2s 6H $CH_3$).
IR (KBr) :
γ $SO_2$-Cl, as 1 365 $cm^{-1}$, s 1 175 $cm^{-1}$ ;
γ$SO_2$-CH<, as 1 300 $cm^{-1}$, s 1 130 $cm^{-1}$.

## Synthèse des médicaments selon l'invention

D'un point de vue pratique, on peut procéder comme suit.

Le sulfohalogénure, par exemple le chlorure de benzènesulfonyle (par exemple environ 0,1 mole) est mis en solution dans par exemple environ 300 ml d'un solvant approprié tel que le chlorure de méthylène, le chloroforme, ou des mélanges éther éthylique/éthanol ou tout autre solvant inerte dans lequel le chlorure de benzènesulfonyle est soluble.

On ajoute ensuite environ 0,3 mole d'une amine tertiaire destinée à accepter l'acide chlorhydrique formé, et de basicité supérieure à celle de l'amino-alcool utilisé dans la suite, tel que la triéthylamine. Puis on ajoute l'amino-alcool goutte à goutte, de préférence en excès par rapport au chlorure de benzènesulfonyle, à raison d'environ 0,1 à 0,12 mole.

Le mélange est maintenu pendant environ 4 heures à température ambiante, puis est évaporé à sec. Le résidu est repris par de l'eau. Les cristaux obtenus sont ensuite séparés, lavés à l'eau, puis recristallisés dans un solvant, par exemple un alcool tel que l'isopropanol, le benzène, ou l'acétate d'éthyle ou un mélange de benzène et d'acétate d'éthyle.

Il est entendu que l'on peut procéder de façon équivalente, si l'on met en œuvre un autre sulfohalogénure, par exemple un bromure de benzènesulfonyle, en lieu et place du susdit chlorure de benzènesulfonyle.

Dans une variante du procédé qui peut être avantageuse, on utilise l'amino-alcool en lieu et place de l'amine tertiaire, comme accepteur de l'acide halogéné formé. Dans ce cas, on met en œuvre environ 2 moles d'amino-alcool par mole de sulfohalogénure.

Lorsque l'un des substituants du noyau aromatique doit être un groupe amine, les médicaments correspondants selon l'invention sont obtenus en réduisant soit par voie chimique (Fe/HCl) soit par réduction catalytique ($H_2$/nickel de Raney) des dérivés nitrés correspondants.

Les exemples qui suivent illustrent le mode de préparation de médicaments dans lequel on a recours au procédé indiqué ci-dessus dans sa généralité.

## Exemple 1

Préparation du chloro-5 N-(hydroxy-2 éthyl) méthoxy-2 nitro-4 benzènesulfonamide (composé n° 281)

A 0,1 mole (28,6 g) de chlorure de chloro-5 méthoxy-2 nitro-4 benzènesulfonyle en solution dans 300 ml de chlorure de méthylène, on ajoute goutte à goutte 0,2 mole (12,2 g ) d'éthanolamine. Le mélange est maintenu 4 heures à température ambiante puis est évaporé à sec. Le résidu est repris par de l'eau. Les cristaux obtenus sont séparés, lavés à l'eau puis recristallisés dans un alcool tel que l'isopropanol.

Rendement = 80 % Point de fusion = 148 °C

Exemple 2

Préparation du chloro-5 N-(hydroxy-2 propyl) méthoxy-2 nitro-4 benzènesulfonamide (composé n° 322)

A 0,1 mole (28,6 g) de chlorure de chloro-5 méthoxy-2 nitro-4 benzènesulfonyle en solution dans 300 ml de chloroforme, on ajoute successivement 20 ml de triéthylamine ou tout autre amine tertiaire dont la basicité est supérieure à celle de l'amino-alcool utilisé, puis 0,1 mole (7,6 g) d'hydroxy-2 propylamine. Le mélange est maintenu 4 heures à température ambiante puis est évaporé à sec. Le résidu est repris par de l'eau. Les cristaux obtenus sont séparés puis lavés abondamment par de l'eau.

Rendement = 80 % Point de fusion = 124 °C

Exemple 3

Préparation de l'isomère L du chloro-5 N-(hydroxy-1 butyl-2) diméthoxy-2,4 benzènesulfonamide (composé n° 1 194)

A 0,015 mole (4,06 g) de chlorure de chloro-5 diméthoxy-2,4 benzènesulfonyle en solution dans 30 ml de chlorure de méthylène, on ajoute 5 ml de triéthylamine puis goutte à goutte, 0,02 mole (1,78 g) d'amino-2 butanol isomère L.

Le mélange est maintenu 4 heures à température ambiante puis est évaporé à sec. Le résidu est repris par de l'eau. Les cristaux obtenus sont séparés à l'eau puis recristallisés dans un mélange benzène acétate d'éthyle.

Rendement = 62 % Point de fusion = 180 °C

$(\alpha)_{436}^{20} = +15{,}8°$ concentration = 1 % dans du méthanol

De la même façon a été obtenu l'isomère L du chloro-5 N(hydroxy-1 butyl-2) méthoxy-2 nitro-4 benzène-sulfonamide.

Point de fusion = 144 °C

$\{\alpha\}^{20} = -14{,}0\,°$ concentration = 1 % dans du méthanol

Exemples 4 à 42

Les substances des exemples 4 à 42, identifiées dans le tableau I suivant, ont été préparées par le même procédé que les substances des exemples 1 à 3, cependant, appliquées aux matières premières appropriées correspondantes.

(Voir Tableau I pages suivantes)

Tableau I : Formule des composés des exemples 4 à 42

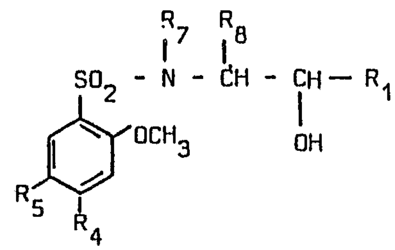

| Composé n° | $R_1$ | $R_4$ | $R_5$ | $R_7$ | $R_8$ | Formule brute | Poids moléculaire | Point de fusion °C | Rendement % |
|---|---|---|---|---|---|---|---|---|---|
| 276 | H | H | Cl | H | H | $C_9H_{12}ClNO_4S$ | 265,72 | 100 | 85 |
| 277 | H | $NO_2$ | H | H | H | $C_9H_{12}N_2O_6S$ | 276,27 | 123 | 78 |
| 282 | H | $NH_2$ | Cl | H | H | $C_9H_{13}ClN_2O_4S$ | 280,73 | 165 | 95 |
| 279 | H | H | $NO_2$ | H | H | $C_9H_{12}N_2O_6S$ | 276,27 | 135 | 75 |
| 278 | H | $NH_2$ | H | H | H | $C_9H_{14}N_2O_4S$ | 246,29 | 170 | 94 |
| 280 | H | H | $NH_2$ | H | H | $C_9H_{14}N_2O_4S$ | 246,29 | 125 | 96 |
| 311 | H | H | Cl | H | $C_2H_5$ | $C_{11}H_{16}ClNO_4S$ | 293,77 | 133 | 74 |

Tableau I (suite)

| Composé n° | $R_1$ | $R_4$ | $R_5$ | $R_7$ | $R_8$ | Formule brute | Poids moléculaire | Point de fusion °C | Rendement % |
|---|---|---|---|---|---|---|---|---|---|
| 318 | $CH_3$ | H | Cl | H | H | $C_{10}H_{14}ClNO_4S$ | 279,74 | 82 | 69 |
| 312 | H | $NO_2$ | H | H | $C_2H_5$ | $C_{11}H_{16}N_2O_6S$ | 304,32 | 139 | 76 |
| 313 | H | $NH_2$ | H | H | $C_2H_5$ | $C_{11}H_{18}N_2O_4S$ | 274,34 | 131 | 95 |
| 319 | $CH_3$ | $NO_2$ | H | H | H | $C_{10}H_{14}N_2O_6S$ | 290,30 | 96 | 81 |
| 320 | $CH_3$ | $NH_2$ | H | H | H | $C_{10}H_{16}N_2O_4S$ | 260,31 | 118 | 96 |
| 314 | H | H | $NO_2$ | H | $C_2H_5$ | $C_{11}H_{16}N_2O_6S$ | 304,32 | 116 | 79 |
| 315 | H | H | $NH_2$ | H | $C_2H_5$ | $C_{11}H_{18}N_2O_4S$ | 274,34 | 139 | 94 |
| 321 | $CH_3$ | H | $NO_2$ | H | H | $C_{10}H_{14}N_2O_6S$ | 290,30 | 136 | 61 |
| 316 | H | $NO_2$ | Cl | H | $C_2H_5$ | $C_{11}H_{15}ClN_2O_6S$ | 338,77 | 145 | 60 |
| 317 | H | $NH_2$ | Cl | H | $C_2H_5$ | $C_{11}H_{17}ClN_2O_4S$ | 308,79 | 164 | 90 |
| 323 | $CH_3$ | $NH_2$ | Cl | H | H | $C_{10}H_{15}ClN_2O_4S$ | 294,76 | 99 | 92 |
| 645 | H | H | Br | H | $C_2H_5$ | $C_{11}H_{16}BrNO_4S$ | 338,21 | 138 | 75 |

Tableau I (suite)

| Composé n° | $R_1$ | $R_4$ | $R_5$ | $R_7$ | $R_8$ | Formule brute | Poids moléculaire | Point de fusion °C | Rendement % |
|---|---|---|---|---|---|---|---|---|---|
| 1 073 | H | $OCH_3$ | $SO_2C_2H_5$ | H | $C_2H_5$ | $C_{14}H_{23}NO_7S_2$ | 381,45 | 157 | 71 |
| 1 074 | $CH_3$ | $OCH_3$ | $SO_2C_2H_5$ | H | H | $C_{13}H_{21}NO_7S_2$ | 367,42 | 139 | 61 |
| 1 075 | H | $OCH_3$ | H | H | $C_2H_5$ | $C_{12}H_{19}NO_5S$ | 289,34 | 122 | 51 |
| 1 163 | $CH_3$ | $OCH_3$ | H | H | H | $C_{11}H_{17}NO_5S$ | 275,31 | 75 | 63 |
| 1 164 | H | $OCH_3$ | $SO_{2n}C_3H_7$ | H | $C_2H_5$ | $C_{15}H_{25}O_7S_2$ | 381,47 | 137 | 66 |
| 1 165 | $CH_3$ | $OCH_3$ | $SO_{2n}C_3H_7$ | H | H | $C_{14}H_{23}O_7S_2$ | 367,44 | 162 | 63 |
| 1 166 | $CH_3$ | $OCH_3$ | $SO_{2i}C_3H_7$ | H | H | $C_{14}H_{23}O_7S_2$ | 367,44 | 181 | 69 |
| 1 167 | H | $OCH_3$ | $SO_{2i}C_3H_7$ | H | $C_2H_5$ | $C_{15}H_{25}O_7S_2$ | 381,47 | 174 | 57 |
| 1 152 | H | $OCH_3$ | $OCH_3$ | H | H | $C_{11}H_{17}NO_6S$ | 291,33 | 149 | 70 |
| 1 153 | H | $OCH_3$ | Cl | H | H | $C_{10}H_{14}ClNO_5S$ | 295,75 | 144 | 72 |
| 1 154 | H | $OCH_3$ | $SO_2C_2H_5$ | H | H | $C_{12}H_{19}NO_7S_2$ | 353,42 | 177 | 80 |
| 1 195 * | H | $NO_2$ | Cl | H | $C_2H_5$ | $C_{11}H_{15}ClN_2O_6S$ | 338,5 | 144 | 40 |

* isomère optique L

Tableau I (suite)

| Composé n° | $R_1$ | $R_4$ | $R_5$ | $R_7$ | $R_8$ | Formule brute | Poids moléculaire | Point de fusion °C | Rendement % |
|---|---|---|---|---|---|---|---|---|---|
| 169 | H | H | Br | $CH_3$ | H | $C_{10}H_{14}BrNO_4S$ | 324 | 88 | 70 |
| 324 | H | H | Cl | $CH_3$ | H | $C_{10}H_{14}ClNO_4S$ | 279,7 | 66 | 82 |
| 325 | H | $NO_2$ | H | $CH_3$ | H | $C_{10}H_{14}N_2O_6S$ | 290,28 | 129 | 74 |
| 326 | H | $NH_2$ | H | $CH_3$ | H | $C_{10}H_{16}N_2O_4S$ | 260,30 | 96 | 72 |
| 327 | H | H | $NO_2$ | $CH_3$ | H | $C_{10}H_{14}N_2O_6S$ | 290,28 | 137 | 66 |
| 328 | H | H | $NH_2$ | $CH_3$ | H | $C_{10}H_{16}N_2O_4S$ | 260,30 | 108 | 90 |
| 329 | H | $NO_2$ | Cl | $CH_3$ | H | $C_{10}H_{13}ClN_2O_6S$ | 324,73 | 108 | 76 |
| 330 | H | $NH_2$ | Cl | $CH_3$ | H | $C_{10}H_{15}ClN_2O_4S$ | 294,75 | 97 | 88 |

Des sulfonamido-alcools conformes à l'invention ont été également obtenus en faisant réagir une amine de formule (XXVI) sur le chlorure de bromo-5 diméthoxy-2,4 benzènesulfonyle.

Les médicaments selon l'invention comprenant à titre de substance active au moins l'un des composés ci-dessus envisagés ainsi que leurs sels physiologiquement acceptables, présentent un ensemble de propriétés pharmacologiques et thérapeutiques de grande valeur.

Parmi les sels physiologiquement acceptables sous lesquels les médicaments selon l'invention peuvent se présenter, on peut notamment citer :

les chlorhydrates, bromhydrates, sulfates, phosphates, méthanesulfonates, tosylates, tartrates, acétates, fumarates, succinate, pyruvate, phénoxyacétate et parachlorophénoxyisobutyrate.

On peut former des sels des composés selon l'invention dans les deux cas suivants :

1) Lorsque $R_7$ est un atome d'hydrogène, on peut former un sel alcalin ou alcalino-terreux physiologiquement acceptable, en utilisant le caractère acide du groupe sulfamoyle.

A titre d'exemple de sels alcalins ou alcalino-terreux, on peut citer les sels de sodium, de potassium, de calcium et de magnésium.

2) Lorsque $R_3$, $R_4$, $R_5$ ou $R_6$ sont des groupes amine, on peut former les sels correspondants tels que les chlorhydrates ou bromhydrates.

Dans le premier cas, on passe du composé au sel en faisant réagir, en quantités équimolaires, et de façon connue en soi, la base et le produit choisis.

Dans le deuxième cas, on passe du composé au sel en faisant réagir, en quantités équimolaires, et de façon connue en soi, l'acide et le produit choisis.

Les médicaments selon l'invention présentent des propriétés remarquables dans le domaine des hyperlipidémies.

## Activité normolipémiante

Les résultats pharmacologiques suivants ont été obtenus avec les composés selon l'invention, sous forme non salifée. Des résultats analogues ont été obtenus avec les sels physiologiquement acceptables de ces composés.

### 1) Tests au Triton

Dans une première série d'expériences, des produits ont été testés sur une hyperlipidémie provoquée par l'administration de TRITON® selon le protocole suivant.

Les essais ont été réalisés sur des groupes de cinq rats WISTAR mâles dont le poids varie de 150 à 175 g.

Les produits testés sont administrés par voie orale, sous forme de solution aqueuse à 3 % de gomme adragante à raison de 1 ml pour 100 g de poids du corps, en quantité correspondant à 250 mg/kg qui est la dose minimale active pour le clofibrate.

Pour effectuer ces essais, on provoque une hyperlipidémie chez les animaux, au moyen du produit commercialisé par la Société ROHM and HAAS sous le nom de TRITON® W. R.

Chez les rats à jeun, l'injection intraveineuse du TRITON® entraîne une hyperlipidémie particulièrement nette pour ce qui est des triglycérides et de façon moins importante pour ce qui est des lipides totaux.

On observe, sur cette hyperlipidémie, les effets obtenus par administration des substances actives selon l'invention.

Les essais sont réalisés comme suit :

les rats sont mis à jeûner pendant 18 heures, puis on administre le TRITON® W.R. par voie intraveineuse en solution aqueuse à 10 % à raison de 200 mg/kg ;

on administre simultanément par voie orale 250 mg/kg de produit à tester ;

6 heures et/ou 24 heures plus tard, on effectue les dosages sur le sang de façon traditionnelle.

Les résultats de ces essais figurent dans le tableau II de la page suivante.

Dans la première colonne, on a indiqué le numéro du composé testé.

Dans la deuxième colonne, on a indiqué le nombre d'animaux qui ont été soumis au test.

Dans les quatre dernières colonnes, on a indiqué les résultats relatifs respectivement :

aux lipides totaux,

aux triglycérides,

au cholestérol total,

au cholestérol estérifié.

Dans chacune de ces colonnes, on indique :

a) le pourcentage de diminution par rapport au témoin TRITON ;

b) le score par rapport au clofibrate de référence, c'est-à-dire le rapport de l'intensité de l'activité du produit testé vis-à-vis de celle de l'activité au clofibrate comptée pour 1.

(Voir Tableau II pages suivantes)

Tableau II : tests au Triton

| Produit | Nombre d'animaux | Lipides totaux | | Triglycérides | | Cholestérol total | | Cholestérol estérifié | |
|---|---|---|---|---|---|---|---|---|---|
| | | % | Score | % | Score | % | Score | % | Score |
| Clofibrate | 5 | 46,2 | 1,00 | 51,6 | 1,00 | 17,7 | 1,00 | 34,0 | 1,00 |
| Composé n° 276 | 5 | 0 | - | 24,8 | 0,48 | 4,4 | 0,25 | 0 | - |
| " 277 | 5 | 0 | - | 19,7 | 0,38 | 3,2 | 0,18 | 2,9 | 0,08 |
| " 278 | 2 | 0 | - | 4,5 | 0,00 | 0 | - | 22,0 | 0,65 |
| " 279 | 4 | 0 | - | 10,2 | 0,20 | 0 | - | 0 | - |
| " 280 | 4 | 0 | - | 0 | - | 1,9 | 0,11 | 2,9 | 0,08 |
| " 281 | 5 | 11,3 | 0,25 | 0 | - | 13,3 | 0,75 | 4,9 | 0,13 |
| " 282 | | 12,0 | 0,26 | 21,6 | 0,42 | 13,3 | 0,75 | 15,5 | 0,46 |
| Clofibrate | 5 | 25,0 | 1,00 | 29,5 | 1,00 | 0 | - | 0 | - |
| Composé n° 311 | 5 | 17,6 | 0,69 | 0 | - | 0 | - | 0 | - |
| " 312 | 5 | 45,4 | 1,79 | 0 | - | 55,1 | - | 54,1 | - |
| " 313 | 5 | 19,4 | 0,77 | 0 | - | 39,3 | - | 39,4 | - |
| " 314 | 5 | 25,9 | 1,02 | 1,2 | 0,04 | 42,1 | - | 44,0 | - |
| Clofibrate | 5 | 13,8 | 1,00 | 72,2 | 1,00 | 9,5 | 1,00 | 49,2 | 1,00 |
| Composé n° 315 | 5 | 49,8 | 3,61 | 38,1 | 0,53 | 17,4 | 1,83 | 0,8 | - |
| " 316 | 5 | 42,6 | 3,09 | 0 | - | 11,1 | 1,17 | 31,2 | 0,63 |
| " 317 | 5 | 42,9 | 3,11 | 0 | - | 1,6 | 0,17 | 25,4 | 0,52 |

Tableau II : tests au Triton (suite)

| Produit | Nombre d'animaux | Lipides totaux | | Triglycérides | | Cholestérol total | | Cholestérol estérifié | |
|---|---|---|---|---|---|---|---|---|---|
| | | % | Score | % | Score | % | Score | % | Score |
| Composé n° 318 | 4 | 10,7 | 0,77 | 0 | - | 14,7 | 1,55 | 32,0 | 0,65 |
| " 319 | 5 | 21,4 | 1,55 | 56,5 | 0,78 | 1,6 | 0,17 | 16,4 | 0,33 |
| " 320 | 5 | 20,5 | 1,48 | 66,1 | 0,91 | 15,3 | 1,61 | 16,7 | 0,34 |
| " 321 | 5 | 39,2 | 2,84 | 76,0 | 1,05 | 13,7 | 1,44 | 46,7 | 0,95 |
| " 322 | 4 | 43,5 | 3,15 | 73,4 | 1,02 | 7,9 | 0,83 | 13,1 | 0,27 |
| Clofibrate | 5 | 44,7 | 1,00 | 0 | - | 9,2 | 1,00 | 43,5 | 1,00 |
| Composé n° 323 | 2 | 55,2 | 1,23 | 53,0 | - | 39,2 | 4,26 | 62,9 | 1,44 |
| " 324 | 5 | 25,9 | 0,58 | 19,4 | - | 26,2 | 2,85 | 22,6 | 0,52 |
| " 325 | 5 | 2,7 | 0,06 | 0 | - | 0 | - | 1,6 | 0,04 |
| " 326 | 5 | 31,9 | 0,71 | 20,3 | - | 3,8 | 0,41 | 29,0 | 0,67 |
| " 327 | 5 | 23,6 | 0,53 | 9,0 | - | 0 | - | 0 | - |
| " 328 | 5 | 20,7 | 0,46 | 0 | - | 0 | - | 0 | - |
| " 329 | 5 | 37,6 | 0,84 | 40,0 | - | 0 | - | 0 | - |
| " 330 | 5 | 0 | - | 0 | - | 0 | - | 41,9 | 0,96 |
| Clofibrate | 5 | 11,7 | 1,00 | 30,4 | 1,00 | 21,2 | 1,00 | 38,9 | 1,00 |
| Composé n° 645 | 5 | 42,3 | 3,62 | 36,8 | 1,21 | 32,6 | 1,54 | 19,4 | 0,50 |

Le clofibrate est utilisé en tant qu'agent normolipémiant classique et est pris dans ces tests comme référence.

Parmi les produits testés, les médicaments présentant des propriétés pharmacologiques remarquables vis-à-vis de la normolipémie,sont ceux appartenant aux groupes définis ci-dessus et qui sont les groupes $G_{17}$ à $G_{28}$.

Les médicaments particulièrement préférés sont rassemblés dans le tableau III ci-après.

Tableau III : Médicaments préférés

| Composé n° | Activité normolipémiante |
|---|---|
| 312 | ++ |
| 313 | + |
| 314 | + |
| 315 | +++ |
| 316 | +++ |
| 317 | +++ |
| 318 | ++ |
| 319 | ++ |
| 320 | ++ |
| 321 | +++ |
| 322 | +++ |
| 323 | ++++ |
| 324 | +++ |
| 326 | + |
| 327 | + |
| 329 | + |
| 645 | +++ |

2) Régime athéromateux

Dans une deuxième série expérimentale, on a testé l'activité de certains produits de l'invention sur des animaux ayant reçu pendant une durée prolongée un régime athéromateux.

L'essai s'effectue sur des rats WISTAR femelles, d'un poids d'environ 160-180 g.

Les animaux reçoivent une alimentation provenant de l'usine d'alimentation nationale de Villemoisson-Sur-Orge, France, et désignée sous la référence UAR A03, enrichie en cholestérol (0,5 %) et en acide cholique (0,5 %).

Après 15 jours de régime athéromateux et tout en maintenant les animaux sous régime athéromateux, on leur administre par voie orale pendant 4 jours 250 000 ui/kg de vitamine $D_2$ commercialisée sous le nom de Stérogyl 25H, en solution dans de l'huile d'olive.

On administre ensuite les produits à étudier pendant 4 semaines, dès la fin de l'administration de l'agent lésionnel.

La quantité de produit administrée aux animaux est de 100 mg/kg.

En fin d'étude, on effectue sur tous les animaux des examens biochimiques (cholestérol total, cholesterol high density lipoprotein (HDL) et cholesterol low density lipoprotein (LDL), et triglycérides).

Autopsie et étude histologique

En fin d'étude, tous les animaux encore vivants sont sacrifiés, et l'on prélève divers organes dont particulièrement l'aorte, pour l'étude histologique de cette dernière. Deux critères sont étudiés : le nombre de zones athéromateuses et l'intensité des lésions.

Le calcul des cotations s'effectue de la façon suivante :

en ce qui concerne l'intensité des lésions, on effectue une cotation allant de 0 pour l'absence de lésion à 54 pour les lésions les plus fortes, et ceci pour chaque type d'altération considéré (augmentation des espaces interlamellaires, interruption des lames élastiques, épaississement des lames élastiques, dépôt de calcium, dépôt de mucopolysaccharides) ; puis on fait l'addition de la totalité des résultats par lot d'animaux ;

en ce qui concerne le nombre de zones athéromateuses, par comptage de ces dernières et addition de la totalité des chiffres obtenus par lot d'animaux.

On calcule ensuite le pourcentage de diminution par rapport aux témoins régime et le score, c'est-à-dire le rapport de l'intensité de l'activité du produit testé vis-à-vis de celle de l'activité du clofibrate, compté pour 1.

Le tableau IV donne les résultats détaillés des examens biochimiques :

a) en % de diminution par rapport au témoin régime ;

b) en score, par rapport au produit de référence, le clofibrate, ce dernier étant compté pour 1.

Le tableau V donne le résultat des examens macroscopiques :

a) en % de diminution des animaux atteints de lésions ;

b) en score par rapport au clofibrate de référence, dont le score est compté pour 1.

Le tableau VI donne les résultats microscopiques avec cotation histologique concernant l'intensité des lésions, et le nombre de zones athéromateuses.

Tableau IV : examens biochimiques

Pourcentage de variation par rapport aux témoins régime et score

| LOT | CHOLESTEROL T | | CHOLESTEROL HDL | | CHOLESTEROL LDL | | TRIGLYCERIDES | |
|---|---|---|---|---|---|---|---|---|
| | % | Score | % | Score | % | Score | % | Score |
| TEM-REGIME | | | | | | | | |
| CLOFIBRATE | - 48,75 | 1,00 | + 104,17 | 1,00 | - 55,37 | 1,00 | - 24,14 | 1,00 |
| IC 77.316 | - 45,73 | 0,94 | - 4,17 | - | - 48,21 | 0,87 | - 31,03 | 1,29 |

Tableau V : examens macroscopiques

| LOT | ANIMAUX ATTEINTS | | |
|---|---|---|---|
| | % | % DIMINUTION / TEM REGIME | SCORE / CLOFIBRATE |
| TEM-REGIME | 100 | | |
| CLOFIBRATE | 81,82 | - 18,18 | 1,00 |
| IC 77.316 | 64,78 | - 35,72 | 1,96 |

(Voir Tableau VI page 31)

Tableau VI : Tests de régime athéromateux : examens microscopiques

| LOT | COTATION HISTOLOGIQUE | | | NOMBRE DE ZONES ATHEROMATEUSES POUR 15 ANIMAUX | | |
|---|---|---|---|---|---|---|
| | Cotation | % Diminution Tém-Régime | Score Clofibrate | Cotation | % Diminution Tém-Régime | Score Clofibrate |
| TEM-REGIME | 132,10 | | | 160,40 | | |
| CLOFIBRATE | 49,10 | - 44,13 | 1,00 | 72,30 | - 54,93 | 1,00 |
| IC 77.316 | 45,00 | - 65,95 | 1,05 | 53,60 | - 66,58 | 1,25 |

La toxicité aiguë est exprimée par la dose létale 50, c'est-à-dire la dose létale entraînant la mort de 50 % des animaux.

Cette étude a été effectuée sur des souris mâles et femelles auxquelles on a administré les produits à tester par voie orale à des concentrations différentes et on a mesuré la létalité sur dix souris mâles et sur 10 souris femelles.

Les essais ont été effectués sur le composé n° 316 dont les résultats sont indiqués dans le tableau VII ci-après.

Tableau VII

| | Souris mâles | Souris femelles |
|---|---|---|
| $DL_0$ | 15 000 mg/kg | ≥ 15 000 mg/kg |
| $DL_{50}$ | 18 500 mg/kg | > 15 000 mg/kg |
| $DL_{100}$ | > 20 000 mg/kg | > 15 000 mg/kg |

On en déduit d'après l'ensemble des résultats que le composé n° 316 est pratiquement dénué de toxicité par voie digestive.

Les médicaments selon l'invention sont avantageusement utilisés à titre de principe actif notamment dans le traitement des maladies du métabolisme des lipides qui sont à la base des manifestations de l'athérosclérose et dont on sait qu'elle correspond à un déséquilibre des lipoprotéines plasmatiques.

Les médicaments selon l'invention ont un très bon indice thérapeutique (rapport dose active/dose toxique). Celui-ci peut être comparé favorablement à celui des substances connues présentant des propriétés de même nature.

En plus des substances actives constituées par les composés de l'invention, les médicaments les contenant peuvent être associés à d'autres substances actives compatibles avec les premières.

Dans ces médicaments selon l'invention, les substances actives sont associées, dans la mesure où cela est nécessaire, avec des excipients et adjuvants traditionnels destinés à faciliter et à améliorer leur utilisation et/ou leur conservation.

En particulier, les substances actives sont associées aux excipients solides ou liquides facilitant leur administration en fonction de la voie d'administration.

Compte tenu de leurs activités et des traitements pour lesquels ils sont utilisés, les médicaments selon l'invention peuvent être administrés par voie parentérale. A cet effet, ils sont présentés sous forme de solutions stériles ou stérilisables, injectables ou propres à être utilisées, pour la préparation extemporanée de solutions injectables. Ces solutions peuvent être présentées sous forme de solutions aqueuses physiologiques, notamment isotoniques d'un de ces composés, telles que les solutions isotoniques salines ou glucosées, les exemples ne présentant bien entendu aucun caractère limitatif quant à la définition des produits physiologiquement acceptables pouvant être utilisés pour former des solutions isotoniques injectables.

Les médicaments selon l'invention peuvent également être administrés par d'autres voies, notamment de suppositoires, ou par voie orale. Administrés par voie orale, ils sont présentés sous des formes

très variées : comprimés, dragées, capsules, gélules, poudres, solutions, suspensions, sirops, ou par voie topique telle que crème, pommade, lotion ou gel.

Dans des présentations pharmaceutiques pour administration par voie parentérale, la dose de produit à administrer, par kilogramme de poids du malade, est comprise d'environ 0,5 à environ 25 mg.

Par voie orale, la dose unitaire est comprise d'environ 10 mg à environ 500 mg, de préférence de 50 mg à 250 mg.

Par voie topique, les présentations comportent de 1 à 20 % en poids de la préparation.

**Revendications** (pour les Etats contractants : BE, CH, DE, GB, IT, LI, LU, NL, SE)

1. Médicament possédant des propriétés normolipémiantes, caractérisé en ce qu'il contient, à titre de substance active, le cas échéant à l'état de sel physiologiquement acceptable, et le cas échéant en association avec un véhicule pharmaceutique, un composé de formule

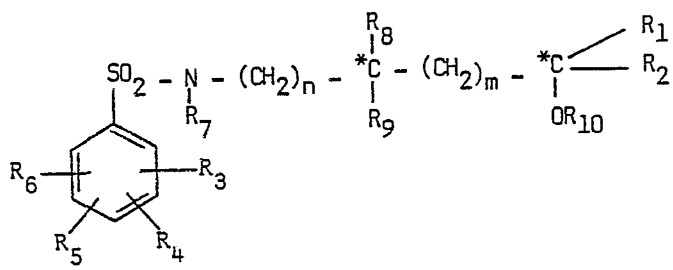

(I)

dans laquelle :

$R_1$, $R_2$ représentent, chacun indépendamment l'un de l'autre :

— un atome d'hydrogène,

— un radical alcoyle inférieur linéaire ou ramifié présentant de 1 à 4 atomes de carbone,

— un radical cycloalcoyle comportant de 3 à 6 atomes de carbone,

— un radical aryle non substitué ou substitué par un groupe nitro, amino, trifluorométhyle, un halogène ou par un reste alcoyle inférieur linéaire ou ramifié contenant de 1 à 4 atomes de carbone, par un reste alcoxy contenant de 1 à 4 atomes de carbone, notamment méthoxy, par un groupe alcoylsulfonyle $—SO_2R$ dans lequel R est un reste alcoyle contenant de 1 à 4 atomes de carbone, par un groupe sulfamoyle $—SO_2NR'R''$ dans lequel R' et R" représentent, indépendamment l'un de l'autre, un atome d'hydrogène ou un reste alcoyle contenant de 1 à 4 atomes de carbone ;

$R_3$, $R_4$, $R_5$ et $R_6$ représentent, indépendamment les uns des autres :

— un atome d'hydrogène,

— un groupe nitro, amino, trifluorométhyle, un halogène,

— un reste alcoyle inférieur linéaire ou ramifié contenant de 1 à 4 atomes de carbone, un reste alcoxy contenant de 1 à 4 atomes de carbone, un groupe alcoylsulfonyle $—SO_2R$ dans lequel R est un reste alcoyle contenant de 1 à 4 atomes de carbone, un groupe sulfamoyle $—SO_2NR'R''$ dans lequel R' et R" représentent, indépendamment l'un de l'autre, un atome d'hydrogène ou un reste alcoyle contenant de 1 à 4 atomes de carbone ;

sous réserve que l'un au moins des substituants $R_3$ à $R_6$ soit différent de l'hydrogène ;

et sous réserve que lorsque l'un des substituants $R_3$ à $R_6$ représente un groupe $NH_2$ en para ou $CH_3$ en para, l'un au moins des autres substituants du noyau aromatique soit différent de l'hydrogène ;

$R_7$, $R_8$ et $R_9$ représentent, indépendamment les uns des autres :

— un atome d'hydrogène,

— un radical alcoyle ayant de 1 à 6 atomes de carbone,

— un radical cycloalcoyle comportant de 3 à 6 atomes de carbone,

— un radical arylalcoyle, notamment phénylalcoyle ou naphtylalcoyle,

dans lequel le radical alcoyle présente de 1 à 4 atomes de carbone, non substitué ou substitué par un groupe nitro, amino, trifluorométhyle, un halogène ou par un reste alcoyle inférieur linéaire ou ramifié contenant de 1 à 4 atomes de carbone, par un reste alcoxy contenant de 1 à 4 atomes de carbone, notamment méthoxy, par un groupe alcoysulfonyle $—SO_2R$ dans lequel R est un reste alcoyle contenant de 1 à 4 atomes de carbone, par un groupe sulfamoyle $—SO_2NR'R''$ dans lequel R' et R" représentent, indépendamment l'un de l'autre, un atome d'hydrogène ou un reste alcoyle contenant de 1 à 4 atomes de carbone ;

$R_{10}$ représente :

— un atome d'hydrogène,

— un groupe acyle comportant de 1 à 12 atomes de carbone ;

n et m varient, indépendamment l'un de l'autre, de 0 à 10, de préférence de 0 à 5, sous réserve que la somme n + m ne dépasse pas 12, de préférence n'excède pas 5.

2. Médicament selon la revendication 1, caractérisé en ce qu'il contient à titre de substance active un composé de formule (I) dans laquelle :

n et m valent 0 ;

$R_9$ représente un atome d'hydrogène ;

et qu'il répond à la formule (II) suivante :

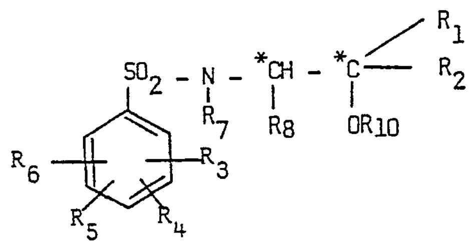

(II)

dans laquelle $R_1$ à $R_8$, $R_{10}$ ont les significations indiquées à la revendication 1, l'un au moins des substituants $R_3$ à $R_6$ étant différent de l'hydrogène.

3. Médicament selon la revendication 2, caractérisé en ce qu'il contient, à titre de substance active, un composé de formule (II) dans laquelle :

$R_1$ et $R_2$ représentent, indépendamment l'un de l'autre :

— un atome d'hydrogène,

— un radical alcoyle inférieur linéaire ou ramifié présentant de 1 à 4 atomes de carbone,

— un radical cycloalcoyle comportant de 3 à 6 atomes de carbone,

— un radical aryle non substitué ou substitué par un groupe nitro, amino, trifluorométhyle, un halogène ou par un reste alcoyle inférieur linéaire ou ramifié contenant de 1 à 4 atomes de carbone, par un reste alcoxy contenant de 1 à 4 atomes de carbone, notamment méthoxy, par un groupe alcoylsulfonyle —$SO_2R$ dans lequel R est un reste alcoyle, contenant de 1 à 4 atomes de carbone, par un groupe sulfamoyle —$SO_2NR'R''$ dans lequel R' et R" représentent, indépendamment l'un de l'autre, un atome d'hydrogène ou un reste alcoyle contenant de 1 à 4 atomes de carbone ;

$R_3$ représente un radical alcoxy inférieur, linéaire ou ramifié présentant de 1 à 4 atomes de carbone, notamment $OCH_3$ ;

$R_4$, $R_5$ et $R_6$ représentent, indépendamment les uns des autres :

— un atome d'hydrogène,

— un groupe $NO_2$, $NH_2$, $CF_3$ ou un atome d'halogène, notamment le chlore ou le brome,

— un groupe alcoylsulfonyle —$SO_2R$, dans lequel R est un reste alcoyle contenant de 1 à 4 atomes de carbone, un reste alcoxy contenant de 1 à 4 atomes de carbone, notamment $OCH_3$, un groupe sulfamoyle —$SO_2NR'R''$ dans lequel R' et R" représentent, indépendamment l'un de l'autre, un atome d'hydrogène ou un reste alcoyle contenant de 1 à 4 atomes de carbone ;

$R_7$ et $R_8$ représentent, indépendamment l'un de l'autre :

— un atome d'hydrogène,

— un radical alcoyle ayant de 1 à 6 atomes de carbone,

— un radical cycloalcoyle comportant de 3 à 6 atomes de carbone,

— un radical arylalcoyle dans lequel le radical alcoyle présente de 1 à 4 atomes de carbone et le groupe aryle est de préférence un groupe phényle non substitué ou substitué par un groupe nitro, amino, trifluorométhyle, un halogène ou par un reste alcoyle inférieur linéaire ou ramifié contenant de 1 à 4 atomes de carbone, par un reste alcoxy contenant de 1 à 4 atomes de carbone ;

$R_{10}$ représente un atome d'hydrogène un groupe acyle, comportant de 1 à 12 atomes de carbone, notamment acétyle, propionyle, phénoxyacétyle, parachlorophénoxyacétyle, pivalyle, adamantyle et embonyle.

4. Médicament selon l'une quelconque des revendications précédentes, caractérisé en ce que, dans la formule de la substance active, $R_3$ est en position 2 et de préférence, consiste en un groupe alcoxy de 1 à 4 atomes de carbone.

5. Médicament selon la revendication 4, caractérisé en ce que la substance active répond à la formule :

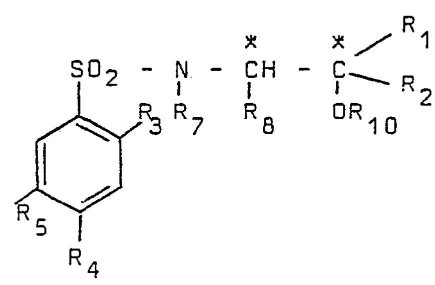

(XII)

dans laquelle :

$R_3$ est un radical alcoxy de 1 à 4 atomes de carbone, notamment méthoxy en position 2,

$R_1$, $R_2$, $R_4$, $R_5$, $R_7$ et $R_8$ présentent les significations sus-indiquées.

6. Médicament selon la revendication 5, caractérisé en ce que la substance active répond à la formule :

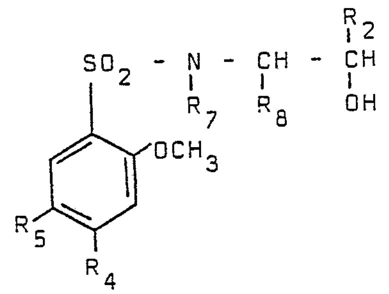

(XVII)

dans laquelle :

$R_2$ représente H ou $CH_3$,

$R_4$ et $R_5$, indépendamment l'un de l'autre, sont choisis dans le groupe comprenant : H, $NO_2$, $NH_2$, Cl, Br, $OCH_3$, $SO_2C_2H_5$, $SO_2iC_3H_7$, $SO_2nC_3H_7$,

$R_7$ représente l'hydrogène ou $CH_3$,

$R_8$ représente H ou $C_2H_5$.

7. Médicament selon la revendication 6, caractérisé ence que $R_4$ et $R_5$ sont choisis dans le groupe comprenant H, Cl, $NO_2$, $NH_2$, Br.

8. Médicament selon la revendication 7, caractérisé en ce que $R_4$ est l'hydrogène et $R_5$ représente Br, Cl, $NO_2$.

9. Médicament selon la revendication 7, caractérisé en ce que $R_4$ représente $NO_2$, $NH_2$, H et $R_5$ est l'hydrogène.

**Revendications** (pour l'Etat contractant AT)

1. Procédé de préparation d'un composé de formule :

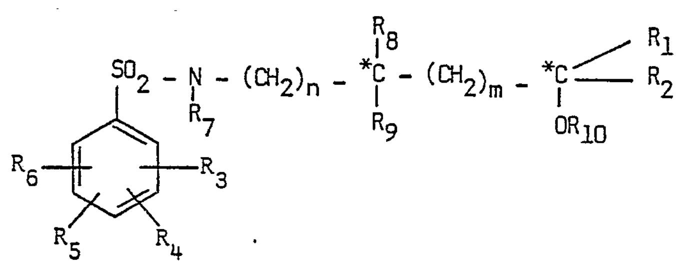

(I)

dans laquelle :

$R_1$, $R_2$ représentent, chacun indépendamment l'un de l'autre :

— un atome d'hydrogène,

— un radical alcoyle inférieur linéaire ou ramifié présentant de 1 à 4 atomes de carbone,

— un radical cycloalcoyle comportant de 3 à 6 atomes de carbone,

— un radical aryle non substitué ou substitué par un groupe nitro, amino, trifluorométhyle, un halogène ou par un reste alcoyle inférieur linéaire ou ramifié contenant de 1 à 4 atomes de carbone, par un reste alcoxy contenant de 1 à 4 atomes de carbone, notamment méthoxy, par un groupe alcoylsulfonyle —$SO_2R$ dans lequel R est un reste alcoyle contenant de 1 à 4 atomes de carbone, par un groupe sulfamoyle —$SO_2NR'R''$ dans lequel R' et R'' représentent, indépendamment l'un de l'autre, un atome d'hydrogène ou un reste alcoyle contenant de 1 à 4 atomes de carbone ;

$R_3$, $R_4$, $R_5$ et $R_6$ représentent, indépendamment les uns des autres :

— un atome d'hydrogène,

— un groupe nitro, amino, trifluorométhyle, un halogène,

— un reste alcoyle inférieur linéaire ou ramifié contenant de 1 à 4 atomes de carbone, un reste alcoxy contenant de 1 à 4 atomes de carbone, un groupe alcoylsulfonyle-$SO_2R$ dans lequel R est un reste alcoyle contenant de 1 à 4 atomes de carbone, un groupe sulfamoyle-$SO_2NR'R''$ dans lequel R' et R'' représentent, indépendamment l'un de l'autre, un atome d'hydrogène ou un reste alcoyle contenant de 1 à 4 atomes de carbone ;

sous réserve que l'un au moins des substituants $R_3$ à $R_6$ soit différent de l'hydrogène ;

et sous réserve que lorsque l'un des substituants $R_3$ à $R_6$ représente un groupe $NH_2$ en para ou $CH_3$ en para, l'un au moins des autres substituants du noyau aromatique soit différent de l'hydrogène ;

$R_7$, $R_8$ et $R_9$ représentent, indépendamment les uns des autres :

— un atome d'hydrogène,
— un radical alcoyle ayant de 1 à 6 atomes de carbone,
— un radical cycloalcoyle comportant de 3 à 6 atomes de carbone,
— un radical arylalcoyle, notamment phénylalcoyle ou naphtylalcoyle,

dans lequel le radical alcoyle présente de 1 à 4 atomes de carbone, non substitué ou substitué par un groupe nitro, amino, trifluorométhyle, un halogène ou par un reste alcoyle inférieur linéaire ou ramifié contenant de 1 à 4 atomes de carbone, par un reste alcoxy contenant de 1 à 4 atomes de carbone, notamment méthoxy, par un groupe alcoylsulfonyle —$SO_2R$ dans lequel R est un reste alcoyle contenant de 1 à 4 atomes de carbone, par un groupe sulfamoyle —$SO_2NR'R''$ dans lequel R' et R'' représentent, indépendamment l'un de l'autre, un atome d'hydrogène ou un reste alcoyle contenant de 1 à 4 atomes de carbone ;

$R_{10}$ représente :
— un atome d'hydrogène,
— un groupe acyle comportant de 1 à 12 atomes de carbone ;

n et m varient, indépendamment l'un de l'autre, de 0 à 10, de préférence de 0 à 5, sous réserve que la somme n + m ne dépasse pas 12, de préférence n'excède pas 5 ;
caractérisé en ce qu'on fait réagir un sulfohalogénure de formule :

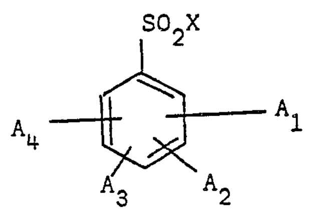

dans laquelle :
— X est un groupe halogène, notamment brome ou de préférence chlore ;
— $A_1$, $A_2$, $A_3$, $A_4$ ont l'une quelconque des significations données pour $R_3$, $R_4$, $R_5$ et $R_6$, à l'exception de $NH_2$ ;
sur une amine de formule :

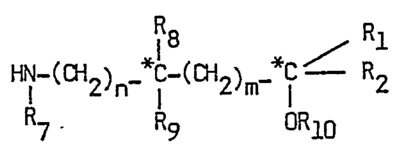

dans laquelle :
n et m ont les significations indiquées ci-dessus :
$R_1$, $R_2$, $R_7$, $R_8$, $R_9$ et $R_{10}$ ont les significations ci-dessus indiquées.

2. Procédé de préparation selon la revendication 1 d'un composé de formule (I), dans laquelle l'un au moins des groupes $R_3$ à $R_6$ représente $NH_2$, caractérisé en ce qu'on hydrogène de façon catalytique le composé de formule (I) dans laquelle l'un des groupes $R_3$ à $R_6$ représente $NO_2$.

3. Procédé de préparation selon l'une quelconque des revendications 1 et 2 d'un composé de formule (I) dans laquelle :
n et m valent 0,
$R_9$ représente un atome d'hydrogène,
et qu'il répond à la formule (II) suivante :

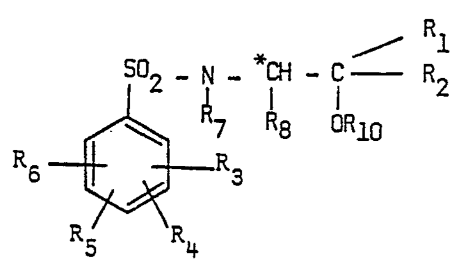

(II)

dans laquelle $R_1$ à $R_8$, $R_{10}$ ont les significations indiquées à la revendication 1, l'un au moins des substituants $R_3$ à $R_6$ étant différent de l'hydrogène.

4. Procédé de préparation selon la revendication 3 d'un composé de formule (II) dans laquelle :
$R_1$ et $R_2$ représentent, indépendamment l'un de l'autre :
— un atome d'hydrogène,
— un radical alcoyle inférieur linéaire ou ramifié présentant de 1 à 4 atomes de carbone,

un radical cycloalcoyle comportant de 3 à 6 atomes de carbone,

un radical aryle non substitué ou substitué par un groupe nitro, amino, trifluorométhyle, un halogène ou par un reste alcoyle inférieur linéaire ou ramifié contenant de 1 à 4 atomes de carbone, par un reste alcoxy contenant de 1 à 4 atomes de carbone, notamment méthoxy, par un groupe alcoysulfonyle $—SO_2R$ dans lequel R est un reste alcoyle contenant de 1 à 4 atomes de carbone, par un groupe sulfamoyle $—SO_2NR'R''$ dans lequel R' et R'' représentent, indépendamment l'un de l'autre, un atome d'hydrogène ou un reste alcoyle contenant de 1 à 4 atomes de carbone ;

$R_3$ représente un radical alcoxy inférieur, linéaire ou ramifié présentant de 1 à 4 atomes de carbone, notamment $OCH_3$ ;

$R_4$, $R_5$ et $R_6$ représentent, indépendamment les uns des autres :

— un atome d'hydrogène,

— un groupe $NO_2$, $NH_2$, $CF_3$ ou un atome d'halogène, notamment le chlore ou le brome,

— un groupe alcoylsulfonyle $—SO_2R$, dans lequel R est un reste alcoyle contenant de 1 à 4 atomes de carbone, un reste alcoxy contenant de 1 à 4 atomes de carbone, notamment $OCH_3$, un groupe sulfamoyle $—SO_2NR'R''$ dans lequel R' et R'' représentent, indépendamment l'un de l'autre, un atome d'hydrogène ou un reste alcoyle contenant de 1 à 4 atomes de carbone ;

$R_7$ et $R_8$ représentent, indépendamment l'un de l'autre :

— un atome d'hydrogène,

— un radical alcoyle ayant de 1 à 6 atomes de carbone,

— un radical cycloalcoyle comportant de 3 à 6 atomes de carbone,

— un radical arylalcoyle dans lequel le radical alcoyle présente de 1 à 4 atomes de carbone et le groupe aryle est de préférence un groupe phényle non substitué ou substitué par un groupe nitro, amino, trifluorométhyle, un halogène ou par un reste alcoyle inférieur linéaire linéaire ou ramifié contenant de 1 à 4 atomes de carbone, par un reste alcoxy contenant de 1 à 4 atomes de carbone ;

$R_{10}$ représente un atome d'hydrogène un groupe acyle, comportant de 1 à 12 atomes de carbone, notamment acétyle, propionyle, phénoxyacétyle, parachlorophénoxyacétyle, pivalyle, adamantyle et embonyle.

5. Procédé de préparation selon l'une quelconque des revendications 1 à 4 d'un composé de formule (I) dans laquelle $R_3$ est en position 2 et, de préférence, consiste en un groupe alcoxy de 1 à 4 atomes de carbone.

6. Procédé de préparation selon la revendication 5 d'un composé de formule :

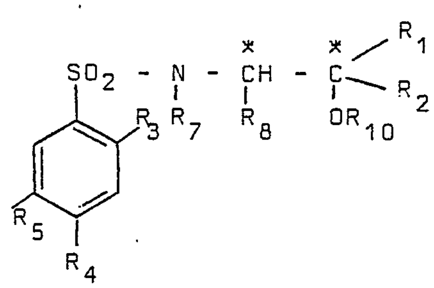

(XII)

dans laquelle :

$R_3$ est un radical alcoxy de 1 à 4 atomes de carbone, notamment méthoxy en position 2 ;

$R_1$, $R_2$, $R_4$, $R_5$, $R_7$ et $R_8$ présentent les significatiosn sus-indiquées.

7. Procédé de préparation selon la revendication 6 d'un composé de formule :

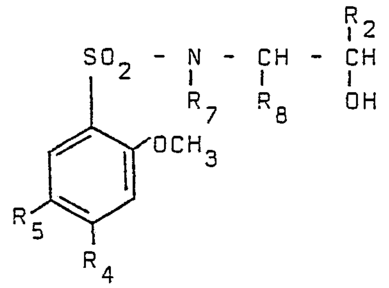

(XVII)

dans laquelle :

$R_2$ représente H ou $CH_3$,

$R_4$ et $R_5$, indépendamment l'un de l'autre, sont choisis dans le groupe comprenant : H, $NO_2$, $NH_2$, Cl, Br, $OCH_3$, $SO_2C_2H_5$, $SO_2iC_3H_7$, $SO_2nC_3H_7$,

$R_7$ représente l'hydrogène ou $CH_3$,

$R_8$ représente H ou $C_2H_5$.

8. Procédé de préparation selon la revendication 7, caractérisé en ce que $R_4$ et $R_5$ sont choisis dans le groupe comprenant H, Cl, $NO_2$, $NH_2$, Br.

9. Procédé de préparation selon la revendication 8, caractérisé en ce que $R_4$ est l'hydrogène et $R_5$ représente Br, Cl, $NO_2$.

10. Procédé de préparation selon la revendication 8, caractérisé en ce que $R_4$ représente $NO_2$, $NH_2$, H et $R_5$ est l'hydrogène.

11. Procédé de préparation d'une composition pharmaceutique caractérisé en ce qu'on associe à un véhicule pharmaceutique, un composé répondant à la formule (I) définie dans la revendication 1 ou telle que limitée dans l'une quelconque des revendications 2 à 10.

**Claims** (for the Contracting States : BE, CH, DE, GB, IT, LI, LU, NL, SE)

1. Medicament possessing lipid-regulating properties, characterized by the fact that it contains, as active substance, if necessary in the state of a physiologically acceptable salt, and if necessary, in association with a pharmaceutical vehicle, a compound of the formula :

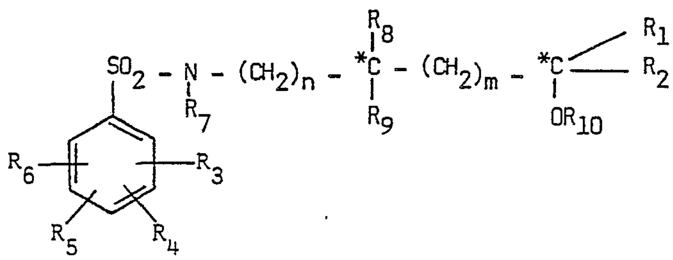

(I)

in which :

$R_1$, $R_2$ represent each independently of one another :

— a hydrogen atom ;

— a linear or branched lower alkyl radical having from 1 to 4 carbon atoms ;

— a cycloalkyl radical comprising from 3 to 6 carbon atoms ;

— an aryl radical, unsubstituted or substituted by a nitro, amino, trifluoromethyl group or halogen atom or by a linear or branched lower alkyl residue having from 1 to 4 carbon atoms, by an alkoxy residue having from 1 to 4 carbon atoms, particularly methoxy, by an alkylsulfonyl group $—SO_2R$ in which R is an alkyl residue having from 1 to 4 carbon atoms, by a sulfamyl group $—SO_2NR'R''$ in which R' and R'' represent, independently of one another, a hydrogen atom or an alkyl residue having from 1 to 4 carbon atoms ;

$R_3$, $R_4$, $R_5$ and $R_6$ represent, independently of one another :

— a hydrogen atom ;

— a nitro group, an amino group, trifluoromethyl group or a halogen ;

— a linear or branched lower alkyl residue having from 1 to 4 carbon atoms, an alkoxy residue having from 1 to 4 carbon atoms, an alkylsulfonyl group $—SO_2R$ in which R is an alkyl residue having from 1 to 4 carbon atoms, a sulfamyl group $—SO_2NR'R''$ in which R' and R'' represent, independently of one another, a hydrogen atom or an alkyl residue having from 1 to 4 carbon atoms ;

provided that when one at least of the substituents $R_3$ to $R_6$ represents a $NH_2$ group at the para position or a $CH_3$ group at the para position, one at least of the other substituents of the aromatic nucleus is different from hydrogen ;

$R_7$, $R_8$ and $R_9$ represent, independently of one another :

— a hydrogen atom ;

— an alkyl radical having 1 to 6 carbon atoms ;

— a cycloalkyl radical comprising from 3 to 6 carbon atoms ;

— an aralkyl radical, particularly phenylalkyl or naphthylalkyl,

in which the alkyl radical has from 1 to 4 carbon atoms, unsubstituted or substituted by a nitro group, an amino group, a trifluoromethyl group, or halogen atom or by a linear or branched lower alkyl residue, by an alkoxy residue having from 1 to 4 carbon atoms, particularly methoxy, by an alkylsulfonyl group, $—SO_2R_2$ in which R is an alkyl residue having from 1 to 4 carbon atoms, by a sulfamyl group —SONR'R'' in which R' and R'' represent, independently of one another, a hydrogen atom or an alkyl residue having from 1 to 4 carbon atoms ;

$R_{10}$ represents :

— a hydrogen atom ;

— an acyl group comprising from 1 to 12 carbon atoms ;

— n and m vary, independently of one another, from 0 to 10, preferably form 0 to 5, provided that the sum n + m does not exceed 12, preferably does not exceed 5.

2. Medicaments according to claim 1, characterized by the fact that they contain as active substance a compound of formula (I) in which :

n and m are equal to 0 ;

$R_9$ represents a hydrogen atom

and complies with the following formula (II) :

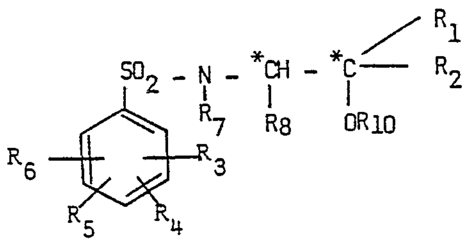

(II)

in which $R_1$ to $R_8$, $R_{10}$ have the above mentioned meanings, one at least of the substituents $R_3$ to $R_6$ being different from hydrogen.

3. Medicaments according to claim 2, characterized by the fact that they contain as active substance a compound of formula (II) in which :

$R_1$ and $R_2$ represent, independently of one another :

— a hydrogen atom ;

— a linear or branched lower alkyl radical having from 1 to 4 carbon atoms ;

— a cycloalkyl radical comprising from 3 to 6 carbon atoms ;

— an aryl radical, unsubstituted or substituted by a nitro group, an amino group a trifluoromethyl group or halogen atom or by a linear or branched lower alkyl residue having from 1 to 4 carbon atoms, by an alkoxy residue having from 1 to 4 carbon atoms, particularly methoxy, by an alkylsulfonyl group —$SO_2R$ in which R is an alkyl residue having from 1 to 4 carbon atoms, by a sulfamyl group —$SO_2NR'R''$ in which R′ and R″ represent independently of one another, a hydrogen atom or an alkyl residue having from 1 to 4 carbon atoms ;

$R_3$ represents a branched or linear alkoxy radical having from 1 to 4 carbon atoms, particularly $OCH_3$ ;

$R_4$, $R_5$ and $R_6$ represent, independently of one another :

— a hydrogen atom ;

— a $NO_2$, $NH_2$, $CF_3$ group or a halogen atom, particularly chlorine or bromine ;

— an alkyl —$SO_2R$ group, in which R is an alkyl residue having from 1 to 4 carbon atoms, an alkoxy residue having from 1 to 4 carbon atoms, particularly $OCH_3$, a sulfamoyl —$SO_2NR'R''$ group in which R′ and R″ represent, independently of one another, a hydrogen atom or an alkyl residue having from 1 to 4 carbon atoms ;

$R_7$ and $R_8$ represent, independently of one another :

— a hydrogen atom ;

— an alkyl radical having from 1 to 6 carbon atoms ;

— a cycloalkyl radical having from 3 to 6 carbon atoms ;

— an arylalkyl radical in which the alkyl radical presents from 1 to 4 carbon atoms and the aryl group is preferably a phenyl group substituted or not by a nitro, an amino or a trifluoromethyl group, a halogen atom or by a linear or branched lower alkyl residue having from 1 to 4 carbon atoms, by an alkoxy residue having from 1 to 4 carbon atoms ;

$R_{10}$ represents a hydrogen atom, an acyl group, having 1 to 12 carbon atoms, particularly acetyl, propionyl, phenoxyacetyl, parachlorophenoxyacetyl, pivalyl, adamantyl and embonyl.

4. Medicament according to anyone of the preceding claims, characterized by the fact that, in the formula of the active substance, $R_3$ is in 2 position and preferably consists of an alkoxy group from 1 to 4 carbon atoms.

5. Medicament according to claim 4, characterized by the fact that the active substance has the formula :

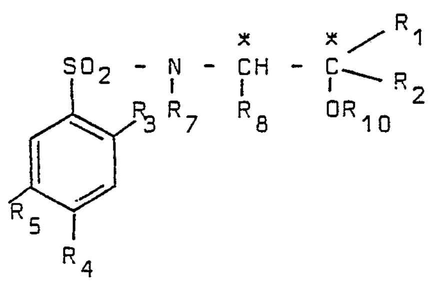

(XII)

in which :

$R_3$ is an alkoxy radical of 1 to 4 carbon atoms, particularly methoxy in 2 position ;

$R_1$, $R_2$, $R_4$, $R_5$, $R_7$ and $R_8$ have the above indicated meanings.

6. Medicament according to claim 5, characterized by the fact that the active substance has the formula :

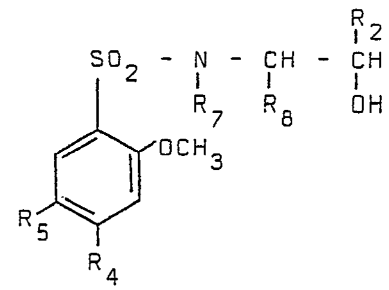

(XVII)

in which :

$R_2$ represents H or $CH_3$ ;

$R_4$ and $R_5$, independently of one another, are chosen among the group comprising : H, $NO_2$, $NH_2$, Cl, Br, $OCH_3$, $SO_2C_2H_5$, $SO_2iC_3H_7$, $SO_2nC_3H_7$ ;

$R_7$ represents hydrogen or $CH_3$ ;

$R_8$ represents H or $C_2H_5$.

7. Medicament according to claim 6, characterized by the fact that $R_4$ and $R_5$ are chosen in the group comprising H, Cl, $NO_2$, $NH_2$, Br.

8. Medicament according to claim 7, characterized by the fact that $R_4$ is hydrogen and $R_5$ represents Br, Cl, $NO_2$.

9. Medicament according to claim 7, characterized by the fact that $R_4$ represents $NO_2$, $NH_2$, H and $R_5$ is hydrogen.

**Claims** (for the Contracting State AT)

1. Process for preparing a compound of formula :

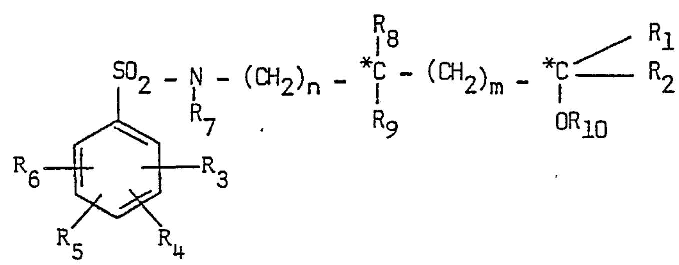

(I)

in which :

$R_1$, $R_2$ each represent independently of one another :

— a hydrogen atom ;

— a linear or branched lower alkyl radical having from 1 to 4 carbon atoms ;

— a cycloalkyl radical comprising from 3 to 6 carbon atoms ;

— an aryl radical, unsubstituted or substituted by a nitro group, an amino group, a trifluoromethyl group, a halogen atom or by a linear or branched lower alkyl residue having from 1 to 4 carbon atoms, by an alkoxy residue having from 1 to 4 carbon atoms, particularly methoxy, by an alkylsulfonyl group $—SO_2R$ in which R is an alkyl residue having from 1 to 4 carbon atoms, by a sulfamoyl group $—SO_2NR'R''$ in which R' and R'' represent, independently of one another, a hydrogen atom or an alkyl residue having from 1 to 4 carbon atoms ;

$R_3$, $R_4$, $R_5$ and $R_6$ represent, independently of one another :

— a hydrogen atom ;

— a nitro, amino, trifluoromethyl group or a halogen ;

— a linear or branched lower alkyl residue having from 1 to 4 carbon atoms, an alkoxy residue having from 1 to 4 carbon atoms, an alkylsulfonyl $—SO_2R$ group in which R is an alkyl residue having from 1 to 4 carbon atoms, a sulfamoyl $—SO_2NR'R''$ group in which R' and R'' represent, independently of one another, a hydrogen atom or an alkyl residue having from 1 to 4 carbon atoms ;

provided that one at least of the substituents $R_3$ to $R_6$ is different from hydrogen ;

and provided that when one at least of the substituents $R_3$ to $R_6$ represents a $NH_2$ group at the para position or a $CH_3$ group at the para position, one at least of the other substituents of the aromatic nucleus is different from hydrogen ;

$R_7$, $R_8$ and $R_9$ represent, independently of one another :

— a hydrogen atom ;

— an alkyl radical having from 1 to 6 carbon atoms ;

— a cycloalkyl radical comprising from 3 to 6 carbon atoms ;

— an aralkyl radical, particularly phenylalkyl or naphthylalkyl,

in which the alkyl radical has from 1 to 4 carbon atoms, unsubstituted or substituted by a nitro, amino, trifluoromethyl, or a halogen or by a linear or branched lower alkyl residue having from 1 to 4 carbon atoms, by an alkoxy residue having from 1 to 4 carbon atoms, particularly methoxy, by an alkylsulfonyl $-SO_2R_2$ group in which R is an alkyl residue having from 1 to 4 carbon atoms, by a sulfamoyl $-SO_2NR'R''$ group in which R' and R'' represent, independently of one another, a hydrogen atom or an alkyl residue having from 1 to 4 carbon atoms ;

$R_{10}$ represents :

— a hydrogen atom ;

— an acyl group comprising from 1 to 12 carbon atoms ;

n and m vary, independently of one another, from 0 to 10, preferably from 0 to 5, provided that the sum n + m does not exceed 12, preferably does not exceed 5 ;

characterized by the fact a sulfohalogenide of formula :

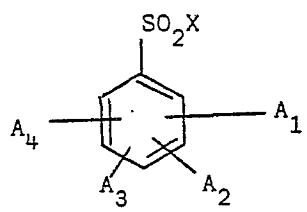

in which :

X is a halogen group, particularly bromine of preferably chlorine ;

$A_1$, $A_2$, $A_3$, $A_4$ have any one of the meanings given for $R_3$, $R_4$, $R_5$ and $R_6$, except $NH_2$ ;

is reacted on an amine of formula :

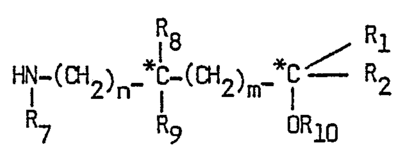

in which :

n and m have the above indicated meanings ;

$R_1$, $R_2$, $R_7$, $R_8$, $R_9$ and $R_{10}$ have the above indicated meanings.

2. Process for preparing, according to claim 1, a compound of formula (I) in which one at least of $R_3$ to $R_6$ groups represents $NH_2$, characterized by the fact that the compound of formula (I) in which one of $R_3$ to $R_6$ groups represents $NO_2$, is hydrogenated according to a catalytic method.

3. Process for preparing, according to anyone of claims 1 and 2, a compound of formula (I) in which :

n and m are equal to 0,

$R_9$ represents a hydrogen atom,

and that it has the following formula (II) :

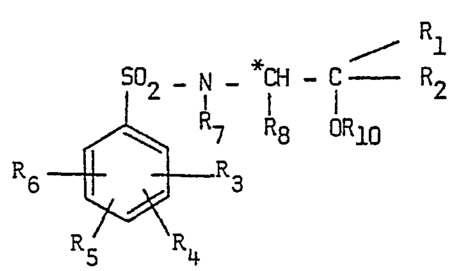

(II)

in which $R_1$ to $R_8$, $R_{10}$ have the meanings indicated in claim 1, one at least of $R_3$ to $R_6$ substituents being different from hydrogen.

4. Process for preparing, according to claim 3, a compound of formula (II) in which :

$R_1$ and $R_2$ represent, independently of one another :

— a hydrogen atom ;

— a linear or branched lower alkyl radical having from 1 to 4 carbon atoms ;

— a cycloalkyl radical having 3 to 6 carbon atoms ;

— an aryl radical substituted or not substituted by a nitro, amino, trifluoromethyl group, a halogen atom or by a linear or branched lower alkyl residue having from 1 to 4 carbon atoms, by an alkoxy residue having from 1 to 4 carbon atoms, notably methoxy, by an alkylsulfonyl $-SO_2R$ group in which R is an alkyl residue having from 1 to 4 carbon atoms, by a sulfamoyl $-SO_2NR'R''$ group in which R' and R'' represent, independently of one another, a hydrogen atom or an alkyl residue having from 1 to 4 carbon atoms ;

$R_3$ represents a linear or branched lower alkoxy radical having from 1 to 4 carbon atoms, notably $OCH_3$ ;

$R_4$, $R_5$ and $R_6$ represent, independently of one another :

— a hydrogen atom ;

— a $NO_2$, $NH_2$, $CF_3$ group or a halogen atom, particularly chlorine or bromine ;

— an alkylsulfonyl $-SO_2R$ group, in which R is an alkyl residue having from 1 to 4 carbon atoms, an alkoxy residue having from 1 to 4 carbon atoms, particularly $OCH_3$, a sulfamoyl $-SO_2NR'R''$ group in which R' and R'' represent, independently of one another, a hydrogen atom or an alkyl residue having from 1 to 4 carbon atoms ;

$R_7$ and $R_8$ represent, independently of one another ;

— a hydrogen atom ;

— an alkyl residue having from 1 to 6 carbon atoms ;

— a cycloalkyl radical having from 3 to 6 carbon atoms ;

— an aralkyl radical in which the alkyl radical has 1 to 4 carbon atoms and the aryl group is preferably a phenyl group substituted or not substituted by a nitro, amino, trifluoromethyl group, a halogen or by a linear or branched lower alkyl residue having from 1 to 4 carbon atoms, by an alkoxy residue having from 1 to 4 carbon atoms ;

$R_{10}$ represents a hydrogen atom, an acyl group having from 1 to 12 carbon atoms, particularly acetyl, propionyl, phenoxyacetyl, parachlorophenoxyacetyl, pivalyl, adamantyl and embonyl.

5. Process for preparing, according to anyone of claims 1 to 4, a compound of formula (I) in which $R_3$ is in the 2 position and, preferably, consists of an alkoxy group of 1 to 4 carbon atoms.

6. Process for preparing according to claim 5 a compound of formula :

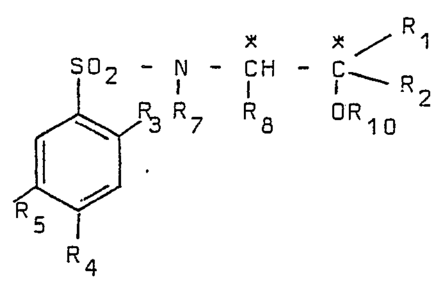

(XII)

in which :

$R_3$ is an alkoxy radical from 1 to 4 carbon atoms, particularly methoxy in the 2 position ;

$R_1$, $R_2$, $R_4$, $R_5$, $R_7$ and $R_8$ have the above indicated meanings.

7. Process for preparing according to claim 6 a compound of formula :

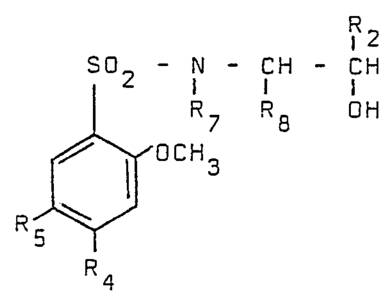

(XVII)

in which :

$R_2$ represents H or $CH_3$ ;

$R_4$ and $R_5$, independently of one another, are chosen in the group comprising : H, $NO_2$, $NH_2$, Cl, Br, $OCH_3$, $SO_2C_2H_5$, $SO_2iC_3H_7$, $SO_2nC_3H_7$ ;

$R_7$ represents hydrogen or $CH_3$ ;

$R_8$ represents H or $C_2H_5$.

8. Process of preparation according to claim 7, characterized by the fact that $R_4$ and $R_5$ are chosen in the group comprising H, Cl, $NO_2$, $NH_2$, Br.

9. Process of preparation according to claim 8, characterized by the fact that $R_4$ is hydrogen and $R_5$ represents Br, Cl, $NO_2$.

10. Process of preparation according to claim 8, characterized by the fact that $R_4$ represents $NO_2$, $NH_2$, H and $R_5$ is hydrogen.

11. Process for preparing a pharmaceutical composition, characterized by the fact that a compound of formula (I) as defined in claim 1 or such as it has been limited in any one of claims 2 to 10, is associated with a pharmaceutical vehicle.

**Patentansprüche** (für die Vertragsstaaten : BE, CH, DE, GB, IT, LI, LU, NL, SE)

1. Normolipämische Eigenschaften aufweisendes Arzneimittel, dadurch gekennzeichnet, daß es als Wirksubstanz, gegebenenfalls in Form eines physiologisch annehmbaren Salzes und gegebenenfalls gemeinsam mit einem pharmazeutischen Träger, eine Verbindung der Formel :

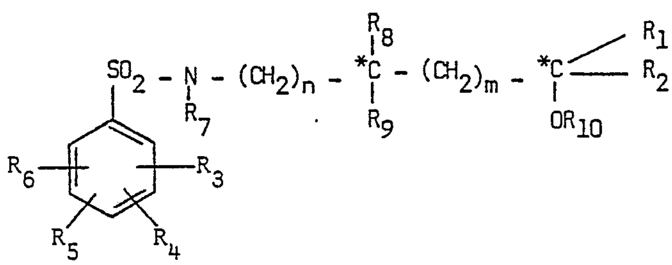

(I)

enthält, in welcher :

$R_1$ und $R_2$ jeweils unabhängig voneinander :

— ein Wasserstoffatom,

— einen niedrigen, geraden oder verzweigten Alkylrest mit 1 bis 4 Kohlenstoffatomen,

— einen Cycloalkylrest mit 3 bis 6 Kohlenstoffatomen,

— einen unsubstituierten Arylrest oder einen Arylrest, der durch eine Nitrogruppe, Aminogruppe, Trifluormethylgruppe, ein Halogen oder durch einen niedrigen, geraden oder verzweigten Alkyrest mit 1 bis 4 Kohlenstoffatomen, durch einen 1 bis 4 Kohlenstoffatome enthaltenden Alkoxyrest, insbesondere Methoxyrest, durch eine Alkylsulfonylgruppe —$SO_2R$, in welcher R ein Alkylrest mit 1 bis 4 Kohlenstoffatomen ist, oder durch eine Sulfamoylgruppe —$SO_2NR'R''$, in welcher R' und R'' unabhängig voneinander ein Wasserstoffatom oder einen Alkylrest mit 1 bis 4 Kohlenstoffatomen bedeuten, substituiert ist ; bedeuten,

$R_3$, $R_4$, $R_5$ und $R_6$ unabhängig voneinander :

— ein Wasserstoffatom,

— eine Nitrogruppe, eine Aminogruppe, eine Trifluormethylgruppe, ein Halogen,

— einen niedrigen, geraden oder verzweigten Alkylrest mit 1 bis 4 Kohlenstoffatomen, einen Alkoxyrest mit 1 bis 4 Kohlenstoffatomen, eine Alkylsulfonylgruppe —$SO_2R$, in welcher R ein 1 bis 4 Kohlenstoffatome enthaltender Alkylrest ist, oder eine Sulfamoylgruppe —$SO_2NR'R''$, in welcher R' und R'' unabhängig voneinander ein Wasserstoffatom oder einen 1 bis 4 Kohlenstoffatome enthaltenden Alkylrest bedeuten ;

mit der Maßgabe, daß wenigstens einer der Substituenten $R_3$ bis $R_6$ von Wasserstoff verschieden ist, und mit der Maßgabe, daß falls einer der Substituenten $R_3$ bis $R_6$ eine $NH_2$-Gruppe in p-Stellung oder eine $CH_3$-Gruppe in p-Stellung bedeutet, wenigstens einer der anderen Substituenten des aromatischen Kerns von Wasserstoff verschieden ist, darstellen,

$R_7$, $R_8$ und $R_9$ unabhängig voneinander :

— ein Wasserstoffatom,

— einen Alkylrest mit 1 bis 6 Kohlenstoffatomen,

— einen 3 bis 6 Kohlenstoffatome enthaltenden Cycloalkylrest oder

— einen Arylalkylrest, insbesondere Phenylalkyl- oder Naphthylalkylrest, bedeuten,

in welchem der Alkylrest 1 bis 4 Kohlenstoffatome aufweist, welcher Alkylrest unsubstituiert oder durch eine Nitrogruppe, eine Aminogruppe, eine Trifluormethylgruppe, ein Halogen oder durch einen niedrigen, geraden oder verzweigten Alkylrest mit 1 bis 4 Kohlenstoffatomen, durch einen 1 bis 4 Kohlenstoffatome enthaltenden Alkoxyrest, insbesondere Methoxyrest, durch eine Alkylsulfonylgruppe —$SO_2R$, in welcher R ein Alkylrest mit 1 bis 4 Kohlenstoffatomen ist, oder durch eine Sulfamoylgruppe —$SO_2NR'R''$, in

welcher R′ und R″ unabhängig voneinander ein Wasserstoffatom oder einen 1 bis 4 Kohlenstoffatome enthaltenden Alkylrest bedeuten, substituiert ist ;

$R_{10}$

— ein Wasserstoffatom oder

— eine 1 bis 12 Kohlenstoffatome enthaltende Acylgruppe bedeutet ;

n und m unabhängig voneinander von 0 bis 10, vorzugsweise von 0 bis 5, mit der Maßgabe variieren, daß die Summe von n + m nicht höher ist als 12, und vorzugsweise 5 nicht überschreitet.

2. Arzneimittel nach Anspruch 1, dadurch gekennzeichnet, daß es als Wirksubstanz eine Verbindung der Formel (I) enthält, in welcher :

n und m gleich 0 sind und

$R_9$ ein Wasserstoffatom bedeutet ;

und welche der folgenden Formel (II) ;

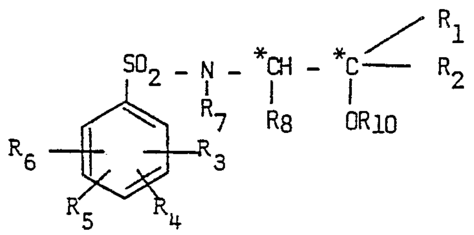

(II)

entspricht, in welcher $R_1$ bis $R_8$ und $R_{10}$ die im Anspruch 1 angegebenen Bedeutungen haben und wenigstens einer der Substituenten $R_3$ bis $R_6$ von Wasserstoff verschieden ist.

3. Arzneimittel nach Anspruch 2, dadurch gekennzeichnet, daß es als Wirksubstanz eine Verbindung der Formel (II) enthält, in welcher :

$R_1$ und $R_2$ unabhängig voneinander :

— ein Wasserstoffatom,

— einen niedrigen, geraden oder verzweigten Alkylrest mit 1 bis 4 Kohlenstoffatomen,

— einen 3 bis 6 Kohlenstoffatome enthaltenden Cycloalkylrest,

— einen unsubstituierten Arylrest oder einen Arylrest, der durch eine Nitrogruppe, eine Aminogruppe, eine Trifluormethylgruppe, ein Halogen oder durch einen niedrigen, geraden oder verzweigten Alkylrest mit 1 bis 4 Kohlenstoffatomen, durch einen 1 bis 4 Kohlenstoffatome enthaltenden Alkoxyrest, insbesondere Methoxyrest, durch eine Alkylsulfonylgruppe —$SO_2R$, in welcher R ein Alkylrest mit 1 bis 4 Kohlenstoffatomen ist, oder durch eine Sulfamoylgruppe —$SO_2NR'R''$, in welcher R′ und R″ unabhängig voneinander ein Wasserstoffatom oder einen 1 bis 4 Kohlenstoffatome enthalten Alkylrest bedeuten, substituiert ist ; darstellen,

$R_3$ einen niedrigen, geraden oder verzweigten Alkoxyrest mit 1 bis 4 Kohlenstoffatomen, insbesondere $OCH_3$, bedeutet ;

$R_4$, $R_5$ und $R_6$ unabhängig voneinander :

— ein Wasserstoffatom,

— eine $NO_2$-Gruppe, eine $NH_2$-Gruppe, eine $CF_3$-Gruppe oder ein Halogenatom, insbesondere Chlor oder Brom,

— eine Alkylsulfonylgruppe —$SO_2R$, in welcher R ein Alkylrest mit 1 bis 4 Kohlenstoffatomen ist, einen Alkoxyrest mit 1 bis 4 Kohlenstoffatomen, insbesondere $OCH_3$, oder eine Sulfamoylgruppe —$SO_2NR'R''$, in welcher R′ und R″ unabhängig voneinander ein Wasserstoffatom oder einen 1 bis 4 Kohlenstoffatome enthaltenden Alkylrest bedeuten, darstellen ;

$R_7$ und $R_8$ unabhängig voneinander :

— ein Wasserstoffatom,

— einen Alkyrest mit 1 bis 6 Kohlenstoffatomen,

— einen 3 bis 6 Kohlenstoffatome enthaltenden Cycloalkylrest,

— einen Arylalkylrest, in welchem der Alkylrest 1 bis 4 Kohlenstoffatome enthält, und die Arylgruppe vorzugsweise eine nicht substituierte Phenylgruppe oder eine Phenylgruppe ist, durch eine Nitrogruppe, eine Aminogruppe, eine Trifluormethylgruppe, ein Halogen oder durch einen niedrigen, geraden oder verzweigten Alkylrest mit 1 bis 4 Kohlenstoffatomen oder durch einen Alkoxyrest mit 1 bis 4 Kohlenstoffatomen substituiert ist ; bedeuten ; und

$R_{10}$ ein Wasserstoffatom oder eine 1 bis 12 Kohlenstoffatome enthaltende Acylgruppe, insbesondere Acetyl, Propionyl, Phenoxyacetyl, p-Chlorphenoxyacetyl, Pivalyl, Adamantyl oder Embonyl, bedeutet.

4. Arzneimittel nach einem der vorhergehenden Ansprüche, dadurch gekennzeichnet, daß $R_3$ in der Formel für die Wirksubstanz in Stellung 2 vorliegt und vorzugsweise eine Alkoxygruppe mit 1 bis 4 Kohlenstoffatomen darstellt.

5. Arzneimittel nach Anspruch 4, dadurch gekennzeichnet, daß die Wirksubstanz der Formel :

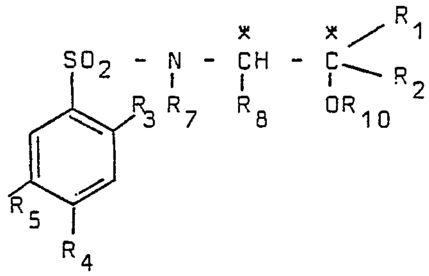

(XII)

entspricht, in welcher:

$R_3$ ein Alkoxyrest mit 1 bis 4 Kohlenstoffatomen, insbesondere Methoxy in Stellung 2, ist und $R_1$, $R_2$, $R_4$, $R_5$, $R_7$ und $R_8$ die oben angegebenen Bedeutungen haben.

6. Arzneimittel nach Anspruch 5, dadurch gekennzeichnet, daß die Wirksubstanz der Formel:

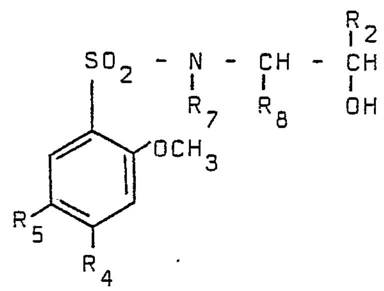

(XVII)

entspricht, in welcher:

$R_2$ H oder $CH_3$ bedeutet,

$R_4$ und $R_5$ unabhängig voneinander aus der H, $NO_2$, $NH_2$, Cl, Br, $OCH_3$, $SO_2C_2H_5$, $SO_2iC_3H_7$ und $SO_2nC_3H_7$ umfassenden Gruppe ausgewählt sind,

$R_7$ Wasserstoff oder $CH_3$ bedeutet, und

$R_8$ H oder $C_2H_5$ bedeutet.

7. Arzneimittel nach Anspruch 6, dadurch gekennzeichnet, daß $R_4$ und $R_5$ aus der H, Cl, $NO_2$, $NH_2$ und Br umfassenden Gruppe ausgewählt sind.

8. Arzneimittel nach Anspruch 7, dadurch gekennzeichnet, daß $R_4$ Wasserstoff ist und $R_5$ Br, Cl oder $NO_2$ darstellt.

9. Arzneimittel nach Anspruch 7, dadurch gekennzeichnet, daß $R_4$ $NO_2$, $NH_2$ oder H darstellt und $R_5$ Wasserstoff ist.

**Patentansprüche** (für den Vertragsstaat AT)

1. Verfahren zur Herstellung einer Verbindung der Formel:

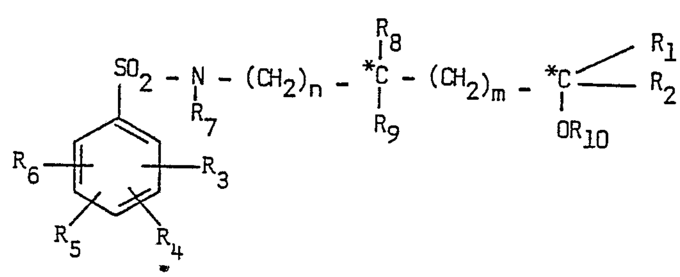

(I),

in welcher:

$R_1$ und $R_2$ jeweils unabhängig voneinander:

— ein Wasserstoffatom,

— einen niedrigen, geraden oder verzweigten Alkylrest mit 1 bis 4 Kohlenstoffatomen,

— einen Cycloalkylrest mit 3 bis 6 Kohlenstoffatomen,

— einen unsubstituierten Arylrest oder einen Arylrest, der durch eine Nitrogruppe, Aminogruppe, Trifluormethylgruppe, ein Halogen oder durch einen niedrigen, geraden oder verzweigten Alkylrest mit 1 bis 4 Kohlenstoffatomen, durch einen 1 bis 4 Kohlenstoffatome enthaltenden Alkoxyrest, insbesondere

Methoxyrest, durch eine Alkylsulfonylgruppe —$SO_2R$, in welcher R ein Alkylrest mit 1 bis 4 Kohlenstoffatomen ist, oder durch eine Sulfamoylgruppe —$SO_2NR'R''$, in welcher R' und R'' unabhängig voneinander ein Wasserstoffatom oder einen Alkylrest mit 1 bis 4 Kohlenstoffatomen bedeuten, substituiert ist ; bedeuten,

$R_3$, $R_4$, $R_5$ und $R_6$ unabhängig voneinander :

— ein Wasserstoffatom,

— eine Nitrogruppe, eine Aminogruppe, eine Trifluormethylgruppe, ein Halogen,

— einen niedrigen, geraden oder verzweigten Alkylrest mit 1 bis 4 Kohlenstoffatomen, einen Alkoxyrest mit 1 bis 4 Kohlenstoffatomen, eine Alkylsulfonylgruppe —$SO_2R$, in welcher R ein 1 bis 4 Kohlenstoffatome enthaltender Alkylrest ist, oder eine Sulfamoylgruppe —$SO_2NR'R''$, in welcher R' und R'' unabhängig voneinander ein Wasserstoffatom oder einen 1 bis 4 Kohlenstoffatome enthaltenden Alkylrest bedeuten ;

mit der Maßgabe, daß wenigstens einer der Substituenten $R_3$ bis $R_6$ von Wasserstoff verschieden ist ; und mit der Maßgabe, daß, falls einer der Substituenten $R_3$ bis $R_6$ eine $NH_2$-Gruppe in p-Stellung oder eine $CH_3$-Gruppe in p-Stellung bedeutet, wenigstens einer der anderen Substituenten des aromatischen Kerns von Wasserstoff verschieden ist ; darstellen,

$R_7$, $R_8$ und $R_9$ unabhängig voneinander :

— ein Wasserstoffatom,

— einen Alkylrest mit 1 bis 6 Kohlenstoffatomen,

— einen 3 bis 6 Kohlenstoffatome enthaltenden Cycloalkylrest oder

einen Arylalkylrest, insbesondere Phenylalkyl- oder Naphthylalkylrest, bedeuten,

in welchem der Alkylrest 1 bis 4 Kohlenstoffatome aufweist, welcher Aralkylrest unsubstituiert oder durch eine Nitrogruppe, eine Aminogruppe, eine Trifluormethylgruppe, ein Halogen oder durch einen niedrigen, geraden oder verzweigten Alkylrest mit 1 bis 4 Kohlenstoffatomen, durch einen 1 bis 4 Kohlenstoffatome enthaltenden Alkoxyrest, insbesondere Methoxyrest, durch eine Alkylsulfonylgruppe —SO R, in welcher R ein Alkylrest mit 1 bis 4 Kohlenstoffatomen ist, oder durch eine Sulfamoylgruppe —$SO_2NR'R''$, in welcher R' und R'' unabhängig voneinander ein Wasserstoffatom oder einen 1 bis 4 Kohlenstoffatome enthaltenden Alkylrest bedeuten, substituiert ist ;

$R_{10}$

— ein Wasserstoffatom oder

— eine 1 bis 12 Kohlenstoffatome enthaltende Acylgruppe bedeutet ;

n und m unabhängig voneinander von 0 bis 10, vorzugsweise von 0 bis 5, mit der Maßgabe variieren, daß die Summe von n + m nicht höher ist als 12, und vorzugsweise 5 nicht überschreitet ;

dadurch gekennzeichnet, daß man ein Sulfonylhalogenid der Formel :

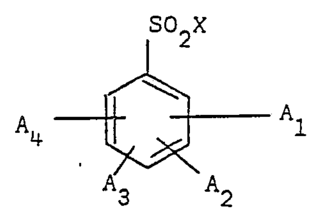

in welcher :

X eine Halogengruppe, insbesondere Brom oder vorzugsweise Chlor ist ; und

$A_1$, $A_2$, $A_3$ und $A_4$ irgendeine der für $R_3$, $R_4$, $R_5$ und $R_6$ angegebenen Bedeutungen, mit Ausnahme von $NH_2$ haben ;

mit einem Amin der Formel :

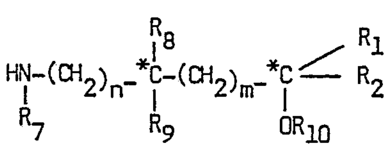

in welcher :

n und m die oben angegebenen Bedeutungen haben und

$R_1$, $R_2$, $R_7$, $R_8$, $R_9$ und $R_{10}$ die oben angegebenen Bedeutungen haben, umsetzt.

2. Verfahren nach Anspruch 1 zur Herstellung einer Verbindung der Formel (I), in welcher wenigstens eine der Gruppen $R_3$ bis $R_6$ $NH_2$ bedeutet, dadurch gekennzeichnet, daß man die Verbindung der Formel (I), in welcher eine der Gruppen $R_3$ bis $R_6$ $NO_2$ bedeutet, katalytisch hydriert.

3. Verfahren nach einem der Ansprüche 1 und 2 zur Herstellung einer Verbindung der Formel (I), in welcher :

n und m gleich 0 sind, und

$R_9$ ein Wasserstoffatom bedeutet ;

und welche der folgenden Formel (II) :

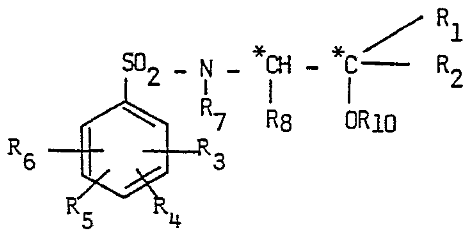

(II)

entspricht, in welcher $R_1$ bis $R_8$ und $R_{10}$ die im Anspruch 1 angegebenen Bedeutungen haben und wenigstens einer der Substituenten $R_3$ bis $R_6$ von Wasserstoff verschieden ist.

4. Verfahren nach Anspruch 3 zur Herstellung einer Verbindung der Formel (II), in welcher

$R_1$ und $R_2$ unabhängig voneinander:

— ein Wasserstoffatom,

— einen niedrigen, geraden oder verzweigten Alkylrest mit 1 bis 4 Kohlenstoffatomen,

— einen 3 bis 6 Kohlenstoffatome enthaltenden Cycloalkylrest,

— einen unsubstituierten Arylrest oder einen Arylrest, der durch eine Nitrogruppe, eine Aminogruppe, eine Trifluormethylgruppe, eine Halogen oder durch einen niedrigen, geraden oder verzweigten Alkylrest mit 1 bis 4 Kohlenstoffatomen, durch einen 1 bis 4 Kohlenstoffatome enthaltenden Alkoxyrest, insbesondere Methoxyrest, durch eine Alkylsulfonylgruppe $—SO_2R$, in welcher R ein Alkylrest mit 1 bis 4 Kohlenstoffatomen ist, oder durch eine Sulfamoylgruppe $—SO_2NR'R''$, in welcher R' und R" unabhängig voneinander ein Wasserstoffatom oder einen 1 bis 4 Kohlenstoffatome enthaltenden Alkylrest bedeuten, substituiert ist; darstellen;

$R_3$ einen niedrigen, geraden oder verzweigten Alkoxyrest mit 1 bis 4 Kohlenstoffatomen, insbesondere $OCH_3$, bedeutet;

$R_4$, $R_5$ und $R_6$ unabhängig voneinander:

— ein Wasserstoffatom,

— eine $NO_2$-Gruppe, eine $NH_2$-Gruppe, eine $CF_3$-Gruppe oder ein Halogenatom, insbesondere Chlor oder Brom,

— eine Alkylsulfonylgruppe $—SO_2R$, in welcher R ein Alkylrest mit 1 bis 4 Kohlenstoffatomen ist, einen Alkoxyrest mit 1 bis 4 Kohlenstoffatomen, insbesondere $OCH_3$, oder eine Sulfamoylgruppe $—SO_2NR'R''$, in welcher R' und R" unabhängig voneinander ein Wasserstoffatom oder einen 1 bis 4 Kohlenstoffatome enthaltenden Alkylrest bedeuten, darstellen;

$R_7$ und $R_8$ unabhängig voneinander:

— ein Wasserstoffatom,

— einen Alkylrest mit 1 bis 6 Kohlenstoffatomen,

— einen 3 bis 6 Kohlenstoffatome enthaltenden Cycloalkylrest,

— einen Arylalkylrest, in welchem der Alkylrest 1 bis 4 Kohlenstoffatome enthält, und die Arylgruppe vorzugsweise eine nicht substituierte Phenylgruppe oder eine Phenylgruppe ist, die durch eine Nitrogruppe, eine Aminogruppe, eine Trifluormethylgruppe, ein Halogen oder durch einen niedrigen, geraden oder verzweigten Alkylrest mit 1 bis 4 Kohlenstoffatomen oder durch einen Alkoxyrest mit 1 bis 4 Kohlenstoffatomen substituiert ist; bedeuten; und

$R_{10}$ ein Wasserstoffatom oder eine 1 bis 12 Kohlenstoffatome enthaltende Acylgruppe, insbesondere Acetyl, Propionyl, Phenoxyacetyl, p-Chlorphenoxyacetyl, Pivalyl, Adamantyl oder Embonyl, bedeutet.

5. Verfahren nach einem der Ansprüche 1 bis 4 zur Herstellung einer Verbindung der Formel (I), in welcher $R_3$ in Stellung 2 vorliegt und vorzugsweise eine Alkoxygruppe mit 1 bis 4 Kohlenstoffatomen darstellt.

6. Verfahren nach Anspruch 5 zur Herstellung einer Verbindung der Formel:

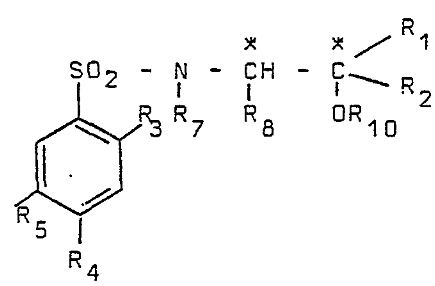

(XII)

in welcher:

$R_3$ ein Alkoxyrest mit 1 bis 4 Kohlenstoffatomen, insbesondere Methoxy in Stellung 2, ist; und

$R_1$, $R_2$, $R_4$, $R_5$, $R_7$ und $R_8$ die oben angegebenen Bedeutungen haben.

7. Verfahren nach Anspruch 6 zur Herstellung einer Verbindung der Formel :

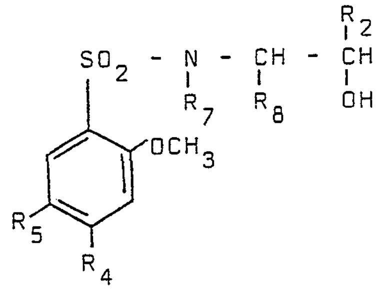

(XVII)

in welcher :

$R_2$ H oder $CH_3$ bedeutet,

$R_4$ und $R_5$ unabhängig voneinander aus der H, $NO_2$, $NH_2$, Cl, Br, $OCH_3$, $SO_2C_2H_5$, $SO_2iC_3H_7$ und $SO_2nC_3H_7$ umfassenden Gruppe ausgewählt sind,

$R_7$ Wasserstoff oder $CH_3$ bedeutet, und

$R_8$ H oder $C_2H_5$ bedeutet.

8. Herstellungsverfahren nach Anspruch 7, dadurch gekennzeichnet, daß $R_4$ und $R_5$ aus der H, Cl, $NO_2$, $NH_2$ und Br umfassenden Gruppe ausgewählt sind.

9. Herstellungsverfahren nach Anspruch 8, dadurch gekennzeichnet, daß $R_4$ Wasserstoff ist und $R_5$ Br, Cl oder $NO_2$ darstellt.

10. Herstellungsverfahren nach Anspruch 8, dadurch gekennzeichnet, daß $R_4$ $NO_2$, $NH_2$ oder H darstellt und $R_5$ Wasserstoff ist.

11. Verfahren zur Herstellung einer pharmazeutischen Zusammensetzung, dadurch gekennzeichnet, daß man einen pharmazeutischen Träger und eine der Formel (I), wie sie in Anspruch 1 definiert ist, oder wie sie in irgendeinem der Ansprüche 2 bis 10 beschränkt ist, entsprechende Verbindung vereinigt.